# NEUE EUROPÄISCHE PATENTSCHRIFT

(11) **EP 1 014 861 B2**
(45) Veröffentlichungstag und Bekanntmachung des Hinweises auf die Entscheidung über den Einspruch: **16.04.2014**
(45) Hinweis auf die Patenterteilung: 31.03.2004
(21) Anmeldenummer: 98954184.2
(22) Anmeldetag: 11.09.1998
(51) Int. Cl.: A61B 10/00

(54) **VORRICHTUNG ZUR ENTNAHME VON BIOLOGISCHEN PROBEN**
DEVICE FOR WITHDRAWING BIOLOGICAL SAMPLES
DISPOSITIF PERMETTANT DE PRELEVER DES ECHANTILLONS BIOLOGIQUES

(30) Priorität: 11.09.1997 DE 19740429
(43) Veröffentlichungstag der Anmeldung: 05.07.2000
(73) Patentinhaber: Caisley International GmbH, 46395 Bocholt (DE)
(72) Erfinder: DITTMANN, Thomas, Claus, D-13409 Berlin (DE); GUT, Ivo, Glynne, D-12161 Berlin (DE); HEUERMANN, Arno, Svend, D-13359 Berlin (DE); OLEK, Alexander, D-10781 Berlin (DE)
(74) Vertreter: Brookhuis, Hendrik Jan Arnold
(86) Internationale Anmeldenummer: PCT/DE1998/002759
(87) Internationale Veröffentlichungsnummer: WO 1999/012475

(56) Entgegenhaltungen:
- EP-A- 0 487 269
- EP-A- 0 684 012
- WO-A-97/19754
- DE-A1- 4 115 031
- DE-A1- 19 707 752
- DE-U1- 8 311 519
- DE-U1- 9 400 043
- FR-A1- 2 696 565
- US-A- 3 731 414
- US-A- 4 651 752
- US-A- 5 461 805
- US-A- 5 643 307
- G. OEHLER ET AL.: 'Schnitt-, Stanz- und Ziehwerkzeuge', Bd. 8, 2001, SPRINGER VERLAG, BERLIN Seiten 19-21, 40-45 - 190-192
- A. GRAW ET AL.: 'Rapid genotyping of low density lipoprotein receptor knockout mice using a polymerase chain reaction technique' LABORATORY ANIMALS Bd. 29, 1995, Seiten 447 - 449

## Beschreibung

### Gebiet der Erfindung

An die Entnahme und Handhabung von biologischen Proben werden sehr unterschiedliche Anforderungen gestellt. Diese sind Menge, Reinheit, Verläßlichkeit und in einigen Fällen sogar die Geschwindigkeit mit der sie genommen werden. Neue, schnelle Analysemethoden machen die Vereinfachung der Probenentnahme notwendig, um eine Senkung der Kosten der Gesamtanalysen zu erreichen. Hohe Anforderungen werden durch die Entnahme von großen Stückzahlen von Proben auch an die Logistik eines solchen Verfahrens gestellt.

Große Mengen von biologischen Analysen sind in der Medizin, Forschung, Züchtung, Qualitätskontrolle und Umwelttechnik von Bedeutung. In allen diesen Bereichen bestimmen folgende Parameter die Anforderungen, die an ein Probenentnahmesystem gestellt werden.
1) die Zahl der zu nehmenden Proben.
2) der Zentralisierungsgrad der Analyse, daß heißt, ob punktuell genommene Proben in großer Menge an einem Ort bearbeitet werden, an dem effizient die Einsortierung in den Analyseprozeß stattfinden muß. Aus einer solchen Situation leitet sich ein anderer Anspruch an die Markierung von Proben ah, als bei einer dezentralisierten Analyse derselben Probenzahl.
3) die Menge der zur Analyse zu verarbeitenden Daten.
4) der Ausbildungsgrad des Personals, welches die Probe entnimmt.
5) der Preis, den eine Analyse kostet, und der Preis, den die Analyse, gemessen am Wert des zu untersuchenden Lebewesens, kosten darf.
6) die Toleranz der Probenentnahme und des Analysesystems gegen Fehler.
7) der Aufwand, mit dem die individuelle Probe entnommen werden muß.
8) die Menge und Gleichmäßigkeit des Probenvolumens, welche pro Probenentnahme entnommen oder abgefüllt werden muß.

Je nach Anwendung werden einige der oben genannten Punkte mehr oder weniger wichtig. Die Erfindung betrifft eine Vorrichtung, welche der Bewältigung der logistischen Probleme bei der Entnahme. Registrierung und Verarbeitung riesiger Probenmengen dient.

US 5 461 805 offenbart eine Ohrmarke mit einem Dornteil und einem Lochteil.

Das Lochteil hat eine Sicherungskappe die das Lösern der Ohrmarke durch Hinter-Lassung sichtbarer Beschadigungen darstellt.

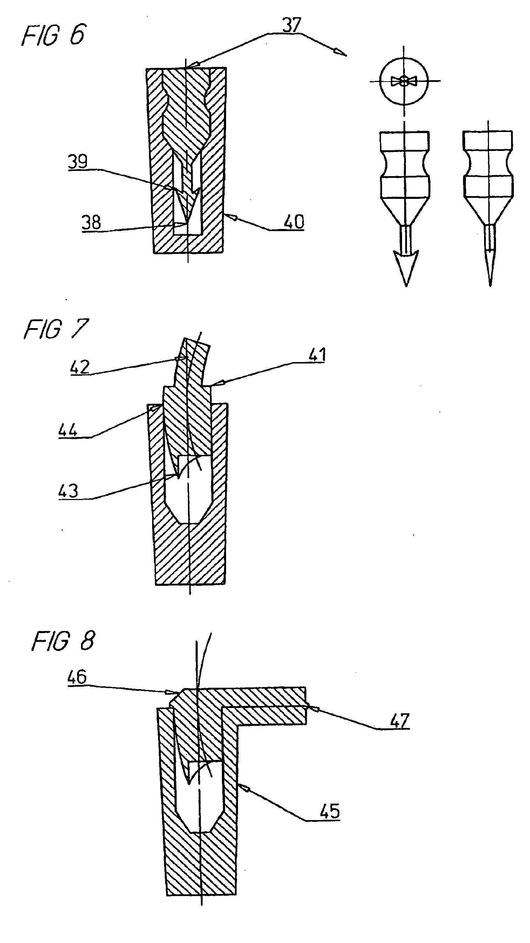

Beim Anbringen der Ohrmarke wird eine biologische Probe des Ohres an der Dornspitze haften. In der Ausbildung nach Fig. 8 kommt diese Probe in eine, von der Sicherungskappe gebildete Kammer.

### Stand der Technik und Aufgaben in der Humanmedizin

Der Stand der Technik bei der Entnahme von medizinischen Proben variiert stark nach Anwendung. Im Prinzip lassen sich Biopsien und einfache Blut-, Urin-, oder Speichelproben unterscheiden.

Bekannt sind diverse, auch automatisierte. Ausführungen von Biopsienadeln (Burbank et al. US Patent 5,526,822), welche aber alle für die Entnahme von wenigen Proben unter schwierigen chirurgischen Bedingungen konstruiert sind. Diese Geräte setzen einen erheblichen Schulungsgrad voraus. Außerdem müssen diese Geräte nach Gebrauch sterilisiert werden und die Proben einzelo verpackt und markiert werden. Mithin sind alle Biopsievorrichtungen nach dem Stand der Technik für andere Anwendungen als das vorliegende Verfahren konstruiert und erfüllen nicht die Ansprüche, welche an dieses gestellt werden.

Bei der Entnahme von Speichel -, Schleim - oder Eiterproben gibt es auch die Variante, daß der eigentliche Probenentnchmer, oft z.B. befestigte Watte oder Gewebebausch direkt im Deckel des zukünftigen Probenbehälters befestigt ist (Holzhäuser et al. DE 32 47 719 A1). Die Probe wird genommen, der Deckel mit dem leeren Behälter verschraubt oder eingerastet, so daß der Watte - oder Gewebebausch im Inneren des Behälters zu liegen kommt. Diese Behälter werden in der Regel mit einem selbstklebenden Barcode versehen.

US 5461805 offenbart eine Ohrmarke mit einem Dornteil und einem Lochteil.

Das Lochteil hat eine Sicherungskappe die das Lösen der Ohrmarke durch Hinterlassung sichtbarer Beschädigungen darstellt.

Beim Anbringen der Ohrmarke wird eine biologische Probe des Ohres an der Dornspitze haften. In der Ausbildung nach Fig. 8 kommt diese Probe in eine, von der Sicherungskappe gebildete Kammer.

### Stand der Technik bei Rindern

Bei der Rinderzucht wird zur gentechnischen Untersuchung, die bis heute nur in geringem Maße durchgeführt wird, eine Gewebeprobe entnommen, indem einige Haarwurzeln des Rindes in einen Probenbehälter gefüllt werden. Der Probenbehälter wird verschlossen und von Hand mit einem Nummerncode oder konkreten Daten beschriftet. Diese Methode stellt keine befriedigende Lösung dar, weil bei großen Viehbeständen der zeitliche Aufwand durch die vielen Arbeitsschritte immens ist. In der Praxis zeigt sich, daß, wenn die Person, welche die Proben entnimmt, nicht direkt mit der Probenanalyse vertraut oder speziell geschult ist, Unregelmäßigkeiten bezogen auf die Probenmenge, Verunreinigungen der Probe oder gar Probenkapseln ohne oder mit falschem Inhalt auftreten können. Milchkühe und die zur Fleischerzeugung gehaltenen Rinder unterliegen unterschiedlichen Bestimmungen, welche wiederholte Standarduntersuchungen vorschreiben. Bei diesen werden unter anderem auch Blutproben durch einen Veterinärmediziner entnommen. Einige spezielle Untersuchungen bedürfen heute keiner Blutprobe und könnten theoretisch problemlos durch eine sehr kleine Gewebeprobe ersetzt werden. Durch ein einfach zu handhabendes Verfahren zur Gewebeentnahme könnten Tierzüchter und Mäster erheblich Tierarztkosten einsparen.

Ab dem I. Januar 1998 wird europaweit die Registrierung von Rindern so vorgenommen, daß jedes Rind innerhalb des ersten Lebensmonats mit zwei Ohrmarken versehen wird,- eine in jedes Ohr. Auf beiden Marken ist das Herkunftsland (z.B. DE für Deutschland, GB für England etc.) des Tieres und eine aus mehreren Ziffern zusammengesetzte Registernummer eingetragen. In machen Fällen wird auch, zusätzlich zur Nummer, ein Barcode verwendet. Die einzige Sicherung vor Verlust und Mißbrauch ist, daß ein Tier jeweils zwei Ohrmarken mit einem identischen Datensatz trägt. Auf Grund der Größe der Ohrmarken reißen diese recht häufig aus, so daß nur noch eine Marke am Tier verbleibt. Eine neue Marke mit dem entsprechenden Code muß vom Viehzuchtverband angefordert werden. Bei Fleckvieh wird eine Zeichnung der Markierung des jeweiligen Tieres erstellt. Diese Zeichnung wird später durch ein Polaroidbild des Rindes ersetzt. Da jedoch hauptsächlich Braunvieh zur Fleischherstellung verwendet wird, entfällt diese zusätzliche Kontrolle in wesentlichen Fällen. Nummern der Ohrmarken und Markierungen werden im Herdbuch des entsprechenden Landesverbandes festgehalten. Es wurden auch Versuche mit subcutan eingepflanzten Transpondern durchgeführt. Diese können sich aber im Verlauf des Lebens eines Rindes im Körper des Tieres bewegen, so daß sie bei der Schlachtung nicht mehr auffindbar sind und in der Wurst enden können.

### Stand der Technik in der Pflanzenzüchtung

Als erste Anwendung wäre hier die Diagnostik von Infektionskrankheiten bei landwirtschaftlich wichtigen Pflanzen zu werten.

Es besteht zur Zeit kein System, welches die Entnahme und eindeutige Markierung der Millionen von Proben und deren zentralisierte Verarbeitung effizient durchzuführen vermag. Heutzutage werden alle Proben manuell, höchstens mit Hilfe von "Zangen" genommen und dann mit einer Pinzette oder von Hand in ein markiertes Gefäß gefüllt. Die Mengen des entnommenen Gewebes sind nicht konstant, so daß jeder Schritt der Probenweiterverarbeitung manuelles Eingreifen erfordert, was zu Fehlern führen kann. Da ein präsymptomatisches Prozedere oft lästig erscheint, besteht die Gefahr, daß auch vorsätzlich falsche Proben genommen werden, wenn kein direkter Nutzen aus einem Ergebnis hervorgeht. Ein Landwirt könnte sogar den Verlust durch angeordnete Vernichtung einer ganzen Ernte befürchten. Dadurch sind die Umstände für vorsätzlichen Betrug gegeben. Soll ein präsymptomatisches System trotzdem eingeführt werden, sind gewisse Kontrollen notwendig. Je billiger ein solches Verfahren sein soll, desto mehr muß eine solche Kontrolle durch automatisierte Technik erfolgen.

Für Züchtungsbemühungen sind riesige Mengen von Probenentnahmen nötig. Bei der Züchtung müssen die Pflanzen gekennzeichnet werden und die Probe muß genau zurückverfolgt werden können. Diese Verfahren erfordern einen enormen organisatorischen Aufwand. Da diese heute noch weitgehend manuell erfolgen, erfordern Züchtungsbemühungen erheblichen Personalaufwand. Dies ist der haupsächliche Kostenfaktor in der Züchtung neuer Sorten.

Die Entwicklung genmanipulierte Pflanzen ist für die Züchter eine enorme Investition. Natürlich will jeder Züchter die Ergebnisse seiner Bemühungen rechtlich gegen unerlaubtes Kopieren absichern. In der Theorie ist die rechtliche Grundlage dafür schon gegeben. Trotzdem beruht ein sicherer Schutz auf Kontrollen. Der Handel mit Saatgut ist global. Es ist vorstellbar, daß riesige Mengen von Stichproben weltweit verschifft werden müssen, um diese Kontrollen durchzuführen. Jede einzelne Probe in einem solchen System muß trotzdem eindeutig ihrer jeweiligen Quelle zuzuordnen sein.

Eine weitere Problematik, welche erst durch die moderne Biotechnologie verursacht wird, ist die Kontrolle von Saatgut und landwirtschaftlichen Produkten auf genetische Manipulationen. Die Konsumenten wünschen in vielen Fällen solche Kontrollen und zur Zeit werden die regulatorischen Mechanismen für solche Kontrollen geschaffen. Auf der technischen Seite bedarf eine solche Kontrolle außer der extrem schnellen und billigen Analyse auch eines hohen Maßes an Logistik hei der Probenentnahme und Registrierung.

Zusammen mit den Entwicklungen auf dem Gebiet der eigentlichen DNA-Analyse erfordert die verbreitete und kostengünstige Anwendung dieser vielversprechenden Techniken eine verbesserte Verfahrensweise, Logistik, Vorrichtungen und ganz generell einen viel höheren Automatisierungsgrad. Das vorliegende Verfahren beinhaltet Vorrichtungen und das detaillierte Konzept für eine Logistik und Datenübertragung und Verwaltung, welche in der Lage ist diese Probleme zu bewältigen. Es wird damit erst möglich die rapide Entwicklung in der Analyse von biologischen Proben durch die notwendige logislische Unterstützung nutzbringend zu verwenden.

Es existieren wenige Verfahren und Vorrichtungen im Gebiet dieser Erfindung. Zwar werden z.B. Rinder registriert, jedoch findet eine gleichzeitige Entnahme einer Gewebeprobe, um davon einen genetischen Fingerabdruck zu erstellen, wodurch die Fälschungssicherheit der Registrierung eklatant erhöht werden könnte, nicht statt. Eine Vorrichtung ist beschrieben worden, mit welcher Biopsien bei Menschen seriell genommen werden können. Was die hier beschriebene Erfindung erst sinnvoll macht, sind neuere Entwicklungen in der Analyse von biologischen Proben. Mittels DNA Massenspektrometric kann eine Einzelanalyse mit sehr großer

Empfindlichkeit in unter einer Sekunde durchgeführt werden. Dadurch werden noch nicht dagewesene Anforderungen an die Leistungsfähigkeit der Probenentnahme und an die Verwaltung von genommen Proben gestellt. Ein Verfahren und Vorrichtungen zur Entnahme von biologischen Gewebeproben kann mit leichten Veränderungen zur Gewebeentnahme beim Menschen oder von Pflanzen eingesetzt werden. Das vorgeschlagene Verfahren beinhaltet Vorrichtungen, welche auf bisher nicht existente Weise diese Probleme lösen können.

Der derzeitige Stand der Technik der Analyse von biologischen Proben erlaubt eine immer effizientere Verarbeitung extrem großer Probenmengen. Moderne medizinische Diagnostik, Gerichtsmedizin, genetisch unterstützte Pflanzenzucht, Qualitätskontrollen biologischen Materials und genetische Stammbaumerstellung von Menschen und Tieren werden in zunehmenden Maße mit riesigen Probenzahlen durchgeführt. Dadurch werden extrem hohe Anforderungen an Probenentnahme und - verwaltung gestellt. Besonders die Entwicklung immer preisgünstigerer DNA Analytik eröffnet Anwendungen, welche bisher aus Kostengründen marktwirtschaftlich uninteressant waren. Dadurch kann sich DNA-Technologie sogar auf landwirtschaftliche Anwendungen ausdehnen. Hier geht es um die Entnahme und datenmäßige Verarbeitung von Millionen von biologischen Proben. Neue Standards zur Vermeidung von Verwechalungen und vorsätzlichem Mißbrauch müssen gesetzt werden. Moderne DNA-Technologien sind derart empfindlich, daß Verunreinigungen ausgeschlossen werden müssen. Durch über die Nahrung auf den Menschen übertragbare Krankheiten ergeben sich neue Anforderungen an die Probenentnahme zur Herkunftskontrolle. Derzeit wird diese Kontrolle oft aus wirtschaftlichen Interessen umgangen. Es existiert kein Verfahren und keine Vorrichtung, welches in der Lage ist die Anforderungen, welche diese neuartigen Anwendungen der Analyse biologischer Proben verlangen, zu erfüllen. Nach dem Stand der Technik ist die Entnahme von Millionen von biologischen Proben unwirtschaftlich. Noch weniger können heutige Vorrichtungen solche Mengen von Proben unter Vermeidung von Kontaminationen entnehmen.

Die Entnahme großer Probenzahlen kann heutzutage nicht unter Ausschluß von Probenverwechslungen durch menschliches Versagen durchgeführt werden. Außerdem erlaubt der Stand der Technik die Registrierung und Verarbeitung aller für riesige Probenzahlen und deren Verarbeitung relevanter Daten nur mit erheblichem personellem Aufwand. Weiterhin beruhen alle existierenden Systeme der Probenentnahme auf dem Willen des Personals diese korrekt durchzuführen und richtige Informationen über jede Probe bereit zu stellen.

Wie im Stand der Technik beschrieben, werden Rinder ab I. Januar 1998 europaweit mit zwei Ohrmarken gekennzeichnet. Ohrmarken können gefälscht werden und es ist mittels dieser Marken nicht festzustellen, ob das schließlich verkaufte Produkt auch von dem Tier stammt, welches in Begleilpapieren angegeben ist. Gerade seit dem Aufkommen der bovinen spongiformen Encephalopathy (BSE, Rinderwahnsinn) wird eine lückenlose Kontrolle der Herkunft von Fleisch gefordert. Leider ist es so, daß fast jedes Glied der Fleischkette (vom Einzelhändler und Konsumenten abgeschen) unter gewissen Bedingungen Interesse daran haben kann die bestehenden Kontrollmechanismen zu umgehen.

### Aufgabe der Erfindung

Es ist die Aufgabe der Erfindung, die in der Beschreibung des Standes der Technik deutlich gewordenen Nachteile auszuräumen. Es soll eine Vorrichtung zur Verfügung gestellt werden, welche eine Entnahme von biologischen Proben in großen Stückzahlen ermöglicht, gleichzeitig eine Verfälschung durch vorsätzlichen Mißbrauch oder Probenverwechsclungen durch menschliches Versagen durch weitgehende Automatisierung der eigentlichen Probenentnahme, sowie die gekoppelte unmittelbare Registrieung der Probendaten mittels der Vorrichtung weitgehend ausschließt. Es ist die Aufgabe der Efindung, zum ersten Mal Vorrichtungen zur Verfügung zu stellen, welche die Entwicklung einer Logistik zur Verarbeitung riesiger Probenzahlen ermöglichen.

### Lösung der Aufgabenstellung

Die Erfindung sieht eine Probenkapsel gemäß Anspruch 18 oder Anspruch 19 vor, wobei sich einer ihrer Bestandteile dazu eignet, direkt als Probenentnehmer zur Entnahme einer biologischen Probe verwendet zu werden. Ein Bestandteil der Probenkapsel wird zur Entnahme einer Gewebeprobe durch Stanzen, Schießen, Kratzen, Kneifen, Stoßen, durch Ausreißen von Haaren oder Ausführen der Bewegung einer Biopsienadel verwendet. Das Laden der Vorrichtung mit den Bestandteilen einer Probenkapsel wird dadurch vereinfacht, daß einer oder mehrere identische Bestandteile solcher Probenkapseln miteinander verkettet sind, die entstehenden Streifen oder Ringe von Teilen von Probenkapseln als Einheiten von mehr als nur einem solchen Teil einer Probenkapsel in das Magazin oder einen analogen Teil einer Vorrichtung geladen werden können, die Richtung, in der diese Streifen oder Ringe mehrerer solcher Einheiten, in die Vorrichtung geladen werden, durch eine an nur einer Stelle eines solchen Streifens oder Ringes angebrachten Markierung festgelegt sind, so daß die Seriennummer aller so verketteten Teile von Prubenkapseln durch nur diese eine Seriennummer in eindeutiger Art und Weise definiert werden. Dies ist natürlich nur nützlich, wenn die Seriennummer, weiche auf diesen Streifen oder Ringen angebracht ist, eine automatisch von einer dafür vorgesehenen Vorrichtung lesbare Form hat oder vom Benutzer selber gelesen und manuell in eine dafür vorgeschene Vorrichtung eingeben werden kann.

Es ist auch für einige Anwendungen notwendig, bei der Entnahme der Probe eine Markierung am Lebewesen anzubringen. Diese Markierung soll zur Fälschungssicherheit beitragen. Die Probenkapsel umfaßt einen oder mehrere Teile, die abgetrennt werden können um als Markierung im Gewebe des Lebewesens zu verbleiben, oder diese Bestandteile der Probenkapsel können nach dem Zusammenfügen der Probenkapsel eine Ohrmarke ergeben, welche nur unter Hinterlassung von sichtbaren Beschädigungen an entweder Ohrmarke oder Lebewesen entfernt werden kann. Außerdem kann der Teil der Probenkapsel, welcher im Gewebe des Lebewesens, dessen Probe entnommen wurde, verbleibt
- einen Barkode.
- einen lesbaren und/oder beschreibbaren integrierten Schaltkreise,
- einen Magnetstreifen,
- einen Transponder,
- einen Sender,
- einen Nummernkode,
- einen Buchstabenkode oder
- eine vergleichbare Kodierungs oder Informationsträgersystem
- oder eine einfache farbliche oder analoge Markierung tragen.

Es wird weiterhin eine Vorrichtung gemäß Anspruch 1 bereitgestellt, welche über eine Aufnahme verfügt, welche in der Lage ist einen oder mehrere Deckel für Probenbehälter (Probenkapseldeckel) aufzunehmen, des weiteren über eine Aufnahme verfügt, welche in der Lage ist einen oder mehrere Probenbehälter aufzunehmen, und über eine Mechanik verfügt, welche den Probenkapseldeckel und den Probenbehälter in einem Arbeitsgang mit der Entnahme einer biologischen Probe entweder durch den Probenkapseldeckel oder den Probenbehälter zur Probenkapsel zusammenfügt.

Die besondere Effizienz des durch die Vorrichtung ausgeführten Entnahmeprozesses wird durch Verwendung automatischer und halbautomatischer Prozesse während der Verwendung geleistet, welche sich wesentlich dadurch auszeichnen, daß diese über eine Mechanik verfügt, welche nach dem Ansetzen der Vorrichtung an ein Gewebe und Betätigung des Auslösemechanismus einen Bestandteil einer Probenkapsel mittels einer Art Schlagholzen derart durch das Gewebe stanzt oder schießt, daß auf dem Wege durch das Gewebe eine Gewebeprobe entnommen wird und der Probenenlnehmer im selben Bewegungsablauf mit einem weiteren Teil einer Probenkapsel zur geschlossenen Probenkapsel zusammengeführt wird. Dies vermeidet jedes manuelle Verschließen, mithin einen kompletten Arbeitsgang. Weiterhin wird der Aufwand zum Beladen der Vorrichtung dadurch minimiert, daß diese ein oder mehr Magazine enthält, in welche jeweils einzelne oder zu Streifen verkettete Probenkapseldeckel, Probenbehälter und Teile von Markierungen geladen werden können und außerdem über eine Mechanik verfügt, welche diese Bestandteile einer Probenkapsel durch Betätigung eines Auslösemechanismus gemeinsam innerhalb der Magazine um eine Position in Richtung eines Probenentnahmemechanismus der Vorrichtung verschiebt. Außerdem werden automatisch durch eine Mechanik die Probenkapseldeckel von einem Magazin aus auf die Achse des Schußbolzens und die Probenbehälter von einem Magazin aus auf die Zielseite der Vorrichtung vorgelegt

Die große Variabilität der Vorrichtung für verschiedenste Anwendungen innerhalb der verschiedenen möglichen Anwendungsmöglichkeiten wird dadurch geleistet, daß die Vorrichtung über einen Mechanismus verfügt, welcher in einem ersten Schritt den probenentnehmenden Teil der Probenkapsel beim Ansetzen der Vorrichtung an das Gewebe und nach Betätigung des Auslösemechanismus derart zusammenpreßt, daß durch eine kneifende Bewegung eine Gewebeprobe entnommen wird, oder in einem ersten Schritt den probenentnehmenden Teil der Probenkapsel beim Ansetzen der Vorrichtung an das Gewebe und nach Betätigung des Auslösemechanismus derart am Gewebe vorbeiführt, daß durch eine kratzende Bewegung eine Gewebeprobe entnommen wird, oder in einem ersten Schritt den probenentnehmenden Teil der Probenkapsel beim Ausetzen der Vorrichtung an das Gewebe und nach Betätigung des Auslösemechanismus derart unter Zusammenquetschen des Probenentnehmers am Gewebe vorbeiführt, daß Haare ausgerissen werden, und dann in einem weiteren Schritt des selben Bewegungsablaufs oder ein nochmaliges Betätigen des Auslösemechanismus den Probenenmehmer mit einem weiteren Teil der Probenkapsel zur Probenkapsel zusammenfügt. Da verschiedenste Arten, und damit Festigkeiten und Stärken von Geweben einer Probenentnahme unterzogen werden müssen, kann der den Probenbehälter führende Teil der Vorrichtung und der den Probenkapseldeckel führende Teil der Vorrichtung derart von entgegengesetzten Seiten fest an ein Gewebe gedrückt werden, daß ein sich auf einer der beiden Seiten befindlicher Mechanismus einen Teil der Probenkapsel zu einer Probenentnahme verwenden kann und der so ausgeüble Druck und der Abstand zwischen dem den Probenbehälter führenden Teil der Vorrichtung und der den Probenkapseldeckel führenden Teil der Vorrichtung durch eine Verstellschraube oder analoge Vorrichtung reguliert werden kann.

Die enormen logistischen Probleme, welche bei der Entnahme, Registrierung, Markierung, Verschickung und Verarbeitung von riesigen Probenmengen entstehen, werden durch eine Reihe bevorzugfer Merkmale gelöst. Zunächst wird die Registrierung entscheidend dadurch vereinfacht, daß die Vorrichtung über eine Möglichkeit zur Dateneingabe und/oder zur Datenausgabe verfügt oder an eine solche gekoppelt ist. Dies kann eine numerische oder alphanumerische Tastatur und/oder eine Einrichtung zum Empfang oder zum Lesen von Daten sein. Daher kann die Vorrichtung über eine Mechanik oder Elektronik, verfügen, mittels derer automatisch Daten wie
- die Seriennummer der Probenbehälter oder mehrerer verketteter Probenbehälter und/oder
- Informationen über das Lebewesen, dessen Probe entnommen wird und/oder
- andere Informationen, welche die Probenentnahme betreffen eingelesen werden.

Über ein Display können eingelesene und von der Vorrichtung bereitgestellte Daten angezeigt werden. Alle so eingegebenen Daten über die Probenkapsel und Lebewesen und alle anderen für die Entnahme oder Verarbeitung relevanten Daten können dann assoziiert werden, dadurch daß die Vorrichtung über eine Möglichkeit verfügt, die eingegebenen oder automatisch eingelesenen Seriennummtern ein Probenkapseln und die eingegebenen oder automatisch eingelesenen Informationen ührr das Lebewesen, dessen Probe entnommen wurde und andere, die jeweilige Probenentnahme betreffende Informationen zu assoziieren und diese Daten samt der Information über die Assoziation dieser Daten auf einem Speichermedium abzulegen.

Der Umgang mit den entnommen Proben wird entscheidend vereinfacht, dadurch, daß das Speichermedium fest in der Vorrichtung installiert ist oder von dieser abtrennbar ist. Dies gestaltet die Wege zur Datenübertragung so flexible, daß allen Anforderungen an Flexibilität und Effizienz zum ersten Mal in genüge getan wird. Im wesentlichen wird dies dadurch erreicht, daß die Vorrichtung eine Elektronik umfaßt mittels derer alle gespeicherten Daten und Informationen über die Assoziation dieser Daten drahtlos übermittelt werden und gegebenenfalls auch Daten drahtlos empfangen werden. Außerdem kann eine Variante der Vorrichtung auch über eine Schnittstelle verfügen, über welche eine Kabelverbindung zwecks Datenübertragung von oder zur Vorrichtung hergestellt werden kann.

Die Vorrichtung ermöglicht die Lösung der Aufgabenstellung dadurch, daß
- eine Probenentnahmevorrichtung mit Bestandteilen einer Probenkapsel geladen wird,
- ein geladener Teil der Probenkapsel eine Gewebeprobe entnimmt,
- der probenentnehmende Teil der Probenkapsel durch die Entnahme oder während oder nach der eigentlichen Entnahme mit mindestens einem anderen Teil der Probenkapsel zu einer fest verschlossenen Einheit, der Probenkapsel, zusammengefügt wird,
- in dieser nachfolgende Verarbeitungsschritte durchgeführt werden können
- eine zur Probenentnahme verwendete Vorrichtung in der Lage ist die Markierung von Probenbehältern entweder selbsttätig zu lesen oder diese Information vom Benutzer entgegenzunehmen und zu verarbeiten,
- Daten über die Identität der zu entnehmenden Probe automatisch oder manuell aufgenommen werden,
- die Daten über die Seriennummern der Probengefäße automatisch von der Entnahmevorrichtung mit den Daten über die Probe assoziiert werden.
- Daten über Probengefäßnummer und Probe auf einem gemeinsamen Speichermedium abgelegt werden.
- die gespeicherten Daten mittels direkter Datenübermittlung vom Entnahmegerät oder eines Zusatzgerätes aus oder durch Transport eines separierbaren Datenträgers zur Analysevorrichtung übermittelt werden.

Innerhalb der Verwendung werden Gewebeprobenentnahmen durch Stanzen, Schießen, Kratzen, Kneifen, Stoßen oder durch Haare ausreißen mit einem Teil oder Teilen einer Probenkapsel durchgeführt. In einer Ausführungsvarinate muß der Benutzer der Vorrichtung vor jeder oder vor einer Reihe von Probenentnahmen Daten eingeben oder bestätigen, um eine Freigabe der Vorrichtung zu erlangen. Dies, gemeinsam mit anderen wesentlichen Schritten des Verfahrens führt dazu, daß alle zur Probenentnahme und Markierung notwendigen Arbeitsschritte in der Art aneinander gekoppelt sind, daß keiner der Arbeitsschritte einzeln ausgeführt werden kann. Dabei führen einige Komponenten des Verfahrens zu einer hohen Sicherheit gegen Fehler oder vorsätzlich falsche Ausführung des Verfahrens. So wird während oder gekoppelt an die eigentliche Probenentnahme eine Markierung des Lebewesens durchgeführt, von dem eine Probe entnommen wird. Außerdem kann die Markierung nach deren Anbringung am Lebewesen nicht oder nur durch Zerstörung oder Beschädigung oder Beschädigung des Lebewesens entfernt werden. Diese kann als Träger entweder
- eines Barkodes.
- eines lesbaren und/oder beschreibbaren integrierten Schaltkreises.
- eines Magnetstreifens.
- eines Transponders.
- eines Senders,
- eines Nummernkodes,
- eines Buchstabenkodes oder
- eines vergleichbaren Kodierungs - oder Informationsträgersystems
oder einer einfachen farblichen Markierung dienen, ein Umstand, der eine nur sehr schwer trennbare, logische und lückenlos dokumentierbare Assoziation zwischen entnommenen Proben und den Lebewesen, deren Probe entnommen wurde, herstellt. Dabei kann die Markierung nicht mehr ohne Beschädigung der Markierung oder des Lebewesens von diesem getrennt werden und die Probe nicht mehr ohne Beschädigung der Probenkapsel manipuliert werden. Damit sind die hohen Ansprüche an die durch den derzeitigen Stand der Technik ungelösten Probleme der Probenentahme. Registrierung und Fälschungssicherheit und bei deren Logistik gelöst.

Die erfindungsgemäße Vorrichtung definiert zum ersten Mal die technische Lösungen für Ansprüche an eine weitgehend fälschungssichere Registrierung von Lebewesen. Die Vorrichtung ist in einzigartiger Weise in der Lage diese Ansprüche auszuführen. Dies läßt sich im auf wesentliche Neuheiten der in der Vorrichtung verwendeten Probenkapseln zurückführen. Zum Beispiel sieht das Verfahren vor, daß die Mechanik zur Auslösung der Vorrichtung durch eine weitere Mechanik blockiert ist, welche nur durch vorherige Eingahe oder Bestätigung von Daten freigegeben wird. Dadurch besteht, durch den von der Vorrichtung mit den Daten durchgeführten Plausibilitätstest ein Zwang zur korrekten Dateneingabe, Gekoppelt an die Probenentnahme kann außerdem eine Markierung am Lebewesen angebracht werden, die entweder aus a) einer einfachen farblichen Markierung b) einer selbstklebenden Folie c) einer haftenden Plakette oder d) einer selbst-haftenden oder fest im Gewebe verankerten Vorrichtung besteht, welche als Träger entweder
- eines Barkodes.
- eines lesbaren und/oder beschreibbaren integrierten Schaltkreises.
- eines Magnetstreifens.
- eines Transponders.
- eines Senders.
- eines Nummernkodes.
- eines Buchstabenkodes oder
- eines vergleichbaren Kodierungs - oder Informationsträgersystems
- oder einer einfachen farblichen Markierung dient.

Damit nicht korrekte Daten eines Lebewesens eingegeben werden, aber darauf hin die Probe eines anderen Lebewesens genommen wird, kann eine Ausführungsvariante über eine Mechanik verfügen, welche die Vorrichtung nur bei gleichzeitigem Empfang des Signals eines Transponders freigibt.

Die Fälschungssicherheit wird dadurch erhöht, daß einzelne oder mehrere in einer Kette zusammengefaßte Probenbehälter über eine Seriennummer verfügen die schon bei der Herstellung oder später angebracht wird. Damit kann jederzeit nachgeprüft werden, ob eine Seriennummer aus dem System verloren geht oder sogar mehrfach auftaucht. Außerdem kann eine Probenkapsel nach dem Verschließen durch den Probenkapseldeckel nur unter Hinterlassen feststellbarer Veränderungen wieder geöffnet oder deren Inhalt erreicht werden. Damit ist Verfälschung oder Austausch der Probe nach einer (Dank der Vorrichtung erzwungenen) korrekten Dateneingabe und Probenentnahme nicht mehr möglich.

Die weitere Verarbeitung der Proben, welche Prozesse umfaßt, in welche die Probenkapseln zwecks höherer Effizienz so nahtlos wie möglich integriert werden müssen wird dadurch erhöht, daß die Probenkapsel eine Stelle aufweisen kann, welche das Durchstoßen mit Nadeln, Bolzen, Kanülen oder vergleichbaren Vorrichtungen z.B. durch ein Septum hindurch erlaubt. Dies erübrigt jedes Öffnen. Varianten der Probenkapsel können auch dadurch gekennzeichnet sein, daß Teile der Probenkapsel mit zur weiteren Verarbeitung der Probe benötigten Reagenzien gefüllt sind. Sollte es innerhalb eines weiteren Verarbeitungsprozesses der entnommenen Proben nötig sein, die Probenkapseln zu öffnen, so kann der Probenkapseldeckel auf der probenabgewandten Seite einen Fortsatz zu dessen leichter Entfernung aus dem Probenbehälter aufweisen, allerdings nicht ohne Spuren an der Kapsel zu hinterlassen.

Generell muß die Probenkapsel mit einigen, durch die Vorrichtung vorgegebenen Eigenschaften ausgestattet sein. Zum Beispiel wird der probenentnehmende Teil der Probenkapsel auf der probenzugewandten Seite einen oder mehrere Schlitze, Bohrungen oder Vertiefungen zur Aufnahme von Gewebe aufweisen. Die folgenden Eigenschaften beschreiben aber nur einige Beispiele von möglichen Ausführungsvarianten. Der Schutzbereich soll allgemeinere und wesentlichere Eigenschaften beinhalten und dadurch durch die folgenden Eigenschaften der Probenkapseln nicht eingeschränkt werden.

Zum Beispiel kann der Probenkapseldeckel als Probennehmer auf der probenabgewandten Seite eine Bohrung, einen Schlitz oder ähnliches zur Fixierung einer Führung umfassen. Eine Ausführungsvariante der Probenkapseln welche derartig ausgeführt sind, daß der probenentnehmende Teil der Probenkapsel auf der der Probe abgewandten Seite Vertiefungen aufweist, welche dazu dienen den Probenentnehmer a) derart an das Gewebe zu pressen und zu quetschen, daß dadurch kneifend eine Gewebeprobe entnommen wird oder b) derart schräg und unter Druck am Gewebe vorbeizuführen, daß kratzend eine Gewebeprobe entnommen wird. Der Probenbehälterdeckel kann aber auch geschlitzt und zum Teil konisch sein, damit zwei oder mehrere Spitzen bei Berührung des Probanden leicht in ihn hineingestoßen werden und dann durch die konische Form zusammengedrückt werden, so daß eine geringe Probenmenge abgeschert bzw. geschnitten wird und kein weiteres Material mehr entnommen wird.

### Vorrichtungsbeispiele

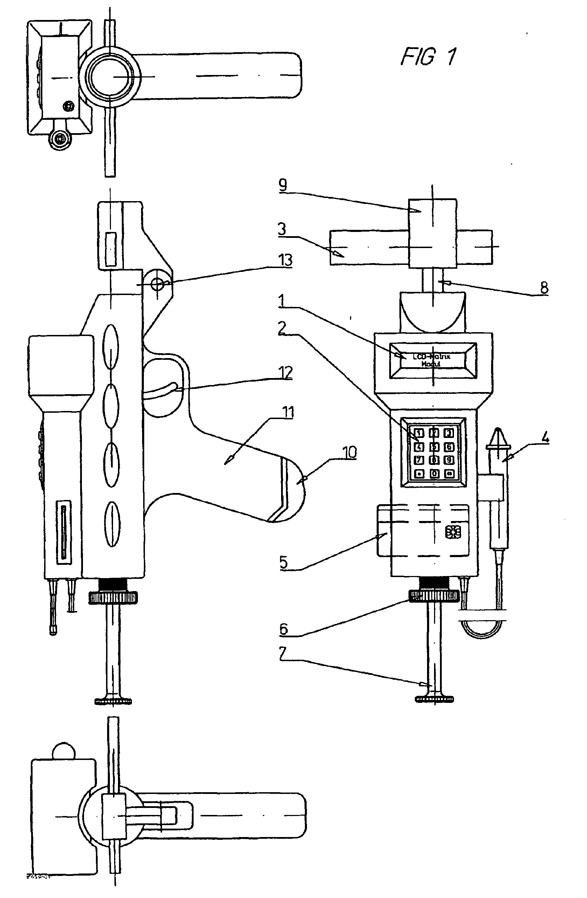

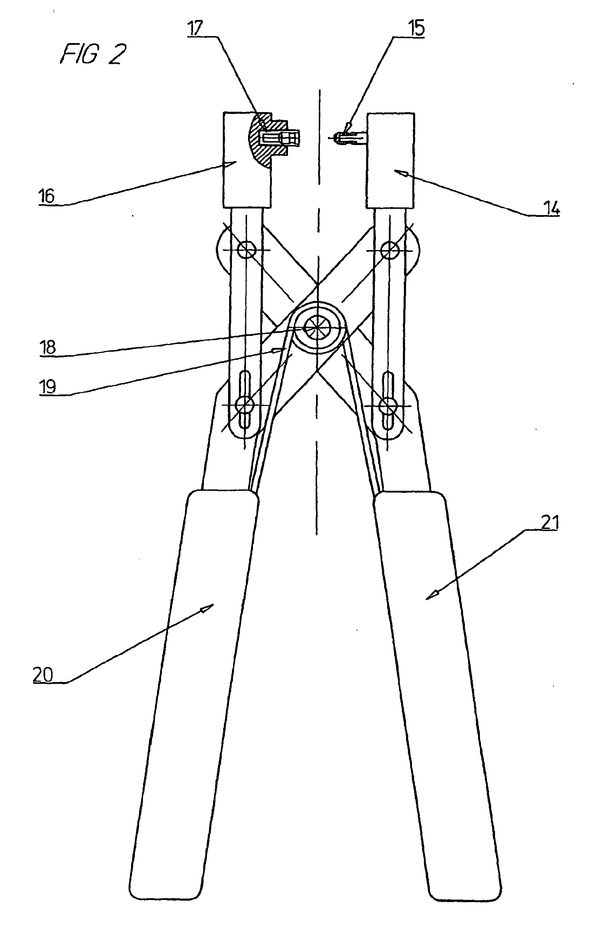

Die Erfindung betrifft Proben kapseln gemäß der Ansprüche 18 oder 19 und Vorrichtungen gemäß Anspruch 1, mit welchen biologische Proben entnommen werden. Gleichzeitig mit der Probenentnahme wird eine Aufnahme der Daten durch eine elektronische Speichereinheit ermöglicht. Mögliche Ausgestaltungen der Vorrichtung zur Probenentnahme sind in Figur 1 und Figur 2 beschrieben. Die Vorrichtungen dienen der Zusammenführung von zwei Bestandteilen einer Probenkapsel (Probenkapseldeckel und Probenbehälter). Die Bestandteile der Probenkapsel sind so ausgeführt, daß der Verschluß der Probenkapsel dazu führt, daß eine Gewebeprobe im Inneren der Probenkapsel zu liegen kommt.

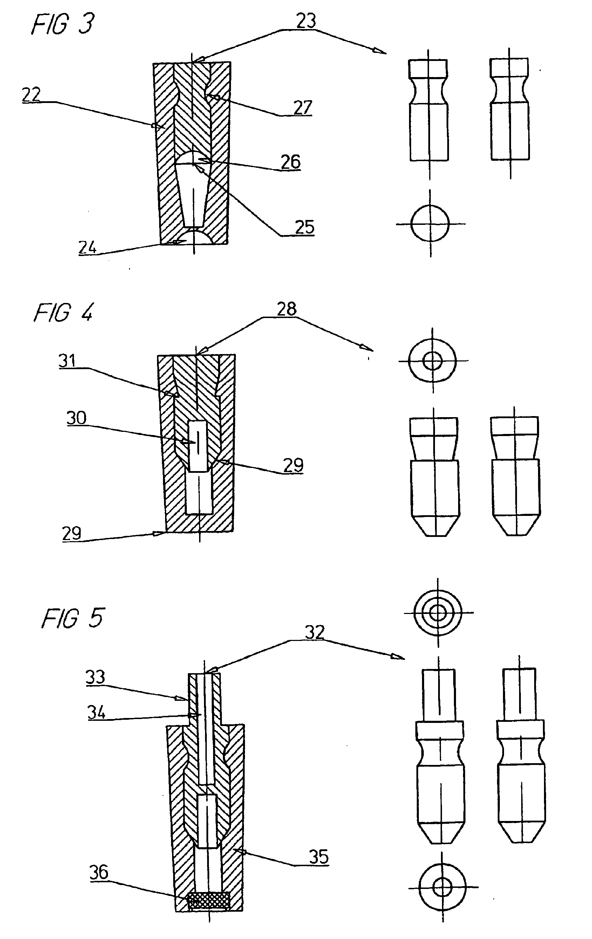

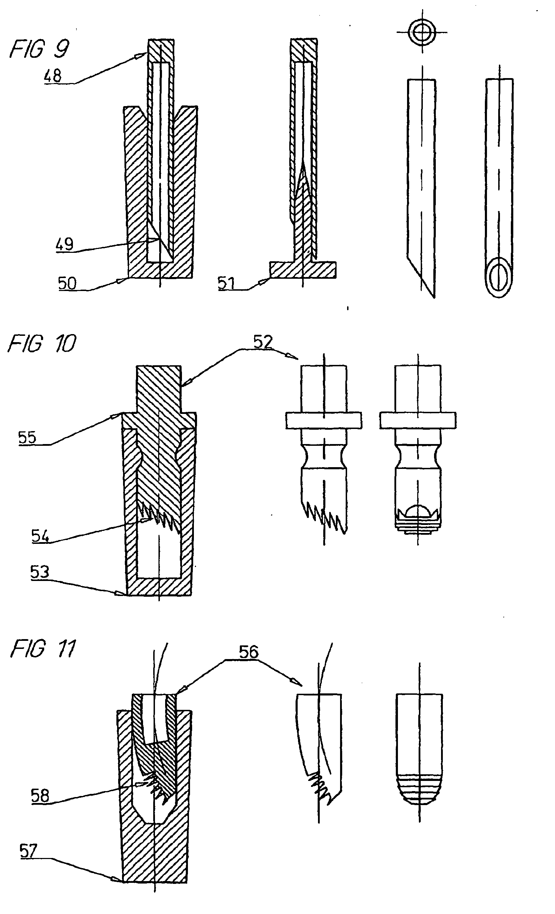

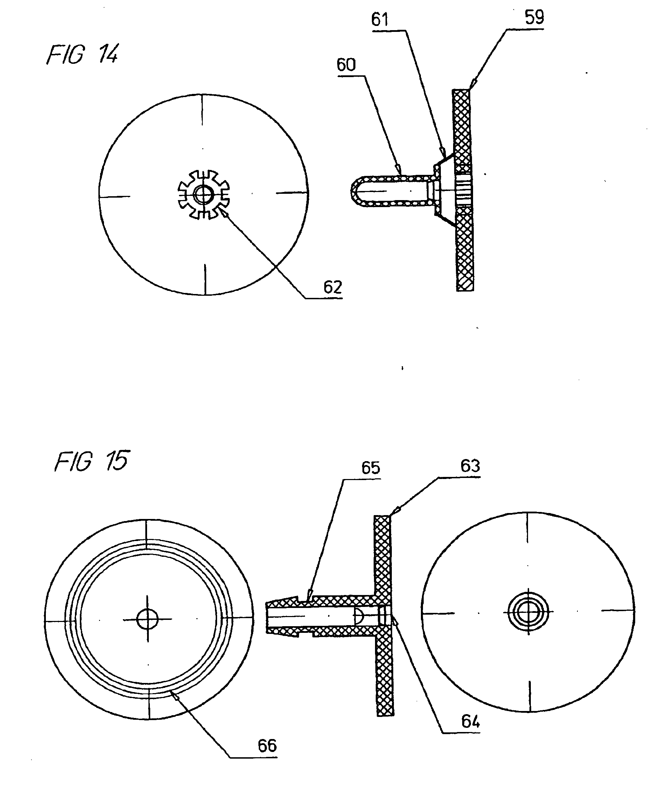

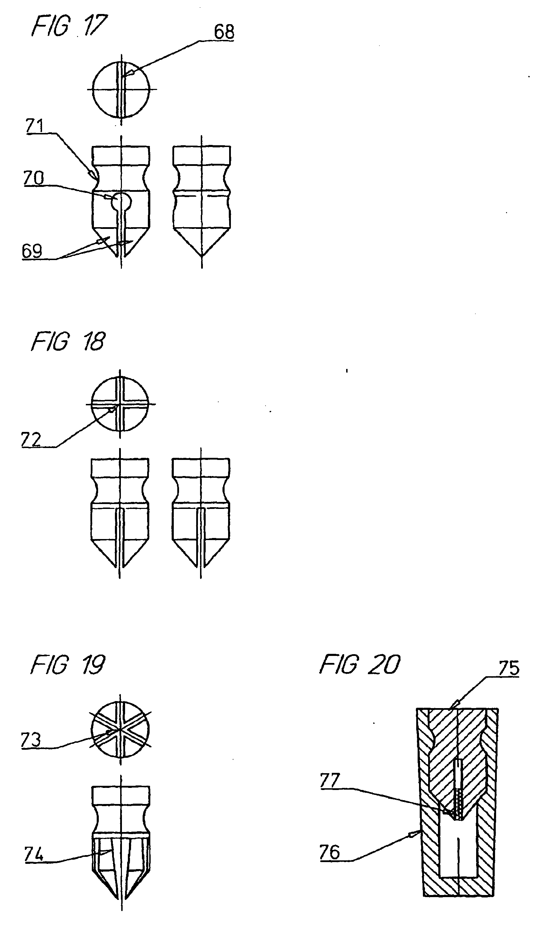

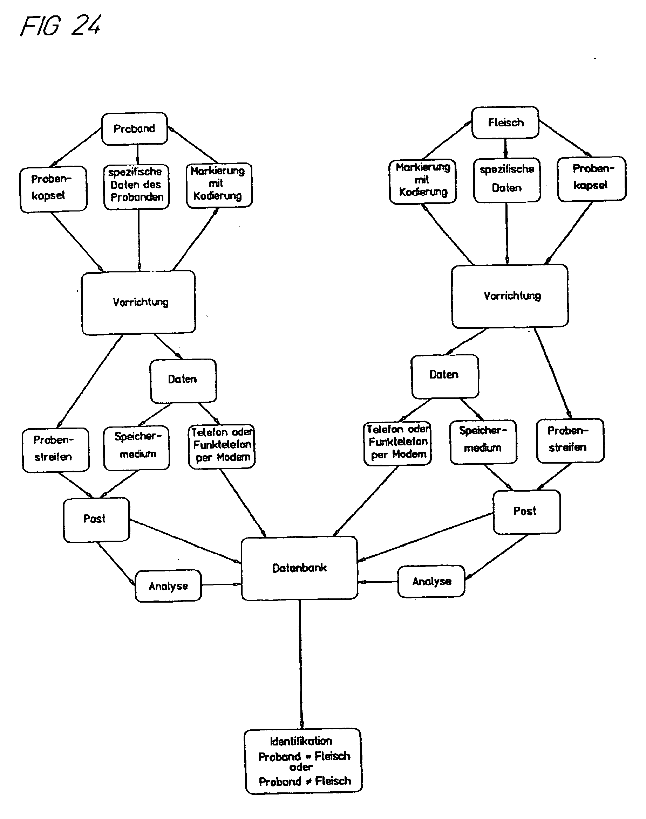

Durch die Eingabe von Daten zur Probe wird jede Probe registriert. Für die Ausführung der Probenkapselbestandteile werden mehrere Varianten vorgeschlagen. Diese Varianten ermöglichen z.B. die Entnahme einer biologischen Probe durch das Ausstanzen einer Gewebeprobe, indem ein Teil der Probenkapsel durch den Organismus hindurchgestoßen wird (Fig. 3-5). Weiter kann ein Probenkapselbestandteil durch den Organismus hindurchgeschoßen werden (Fig. 17-20). Eine weitere Variante ist, daß die Probenkapselbestandleil eine Gewebeprobe von einer Oberfläche abkratzen, die anschließend in der Probenkapsel zu liegen kommt (Fig. 10+11). Ein Gewebeprobe kann auch abgekniffen werden (Fig. 7+8). Varianten, die ähnlich wie eine Biopsienadel funktionieren sind auch möglich (Fig. 6+9). Eine bevorzugte Variante bezieht sich auf die Registrierung von Rindern, weshalb ein gleichzeitiges Anbringen einer Ohrmarke mit der Entnahme einer biologischen Probe, zur Erstellung eines genetischen Fingerabdrucks, durchgeführt wird (Fig. 14-16, 25-26). In Figur 24 wird beschrieben, wie die Kontrolle einer Fleischprobe stattfinden kann, indem die Resultate eines genetischen Fingerabdrucks der Fleischprobe mit dem genetischen Fingerabdruck des Tieres verglichen werden.

Die Vorrichtungen ermöglichen die Probenentnahme in sehr rascher Folge. Da die Bestandteile einer Probenkapsel nur einmal verwendet werden, treten keine Probleme mit Verunreinigungen auf und es besteht kein Bedarf der Sterilisation. Dazu kommt die Betrugssicherheit des Systems, indem die Entnahme einer Probe mit der unmittelbaren Eingabe von Daten zum entsprechenden Lebewesen gekoppelt ist. Durch die automatische Entnahme von biologischen Proben und der gleichzeitigen Registrierung wird ein unvergleichlich schnelle Entnahme der Proben erreicht. Dies eröffnet auch Möglichkeiten in der Verknüpfung mit sehr schnellen Analysemethoden. Es ist denkbar, daß eine Variante der Vorrichtung, wie sie in Fig. 1 gezeigt ist, in der Notfallmedizin und im Operationssaal zum Einsatz kommen kann, um Gewebeproben schnell einer Laboranalyse zuführen zu können. Andere Anwendungen, die ein einfache und dadurch kostengünstige Probenentnahme brauchen, finden wir in der Pflanzenzüchtung und -qualitätskontrolle. Eine Ausführung der Vorrichtung in Figur 1 wird für das Verfahren angewendet, um biologische Gewebeproben aus Blättern zu entnchmen und zu registrieren.

Fig. 1 Ein technisches Beispiel für eine mechanisch vollautomatische arbeitende Vorrichtung zur Probenentnahme in größeren Stückzahlen. Die Vorrichtung besteht im wesentlichen aus einer mechanischen und einer elektronischen Einheit. Die Mechanik der Vorrichtung beruht auf dem Prinzip des Schießens bzw. des Stechens oder Stanzens, wobei der eigentliche Vorteil dadurch gekennzeichnet ist. daß die Probe mit dem Probendeckel entnommen wird und im gleichen Arbeitsgang mit dem Probenbehälter zu einer fest verschlossenen Probenkapsel verbunden wird.

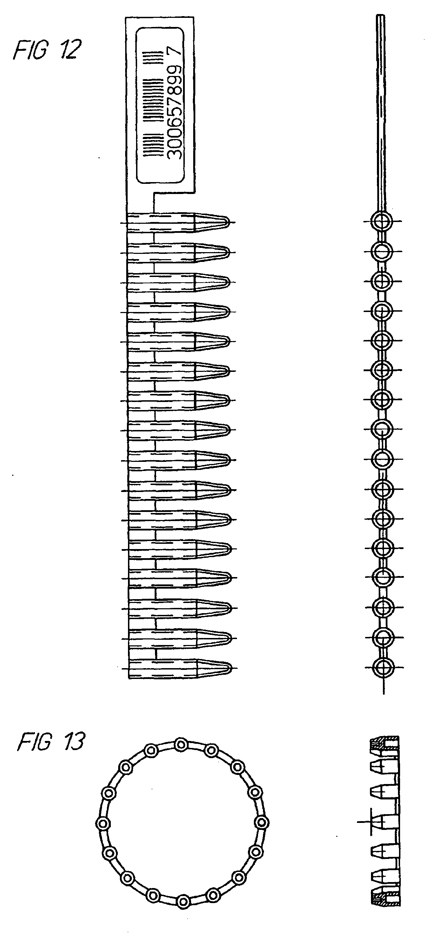

Diese Art der Probenentnahme ermöglicht eine absolut verunreinigungsfreie, sowie bei lebenden Probanden gleichzeitig eine sterile Entnahme. Die hier als Beispiel angeführte Vorrichtung soll zur Probenentnahme bei Tieren eingesetzt werden. Hier wird die Gewebeprobe beim Durchstechen des Ohrrandes entnommen. Die Vorrichtung besitzt einen Schußbolzen 7 der seine Schußkraft über eine in der Vorrichtung befindliche Schußfeder erhält. Die Schußkraft der Feder läßt sich durch die Rändelmutter 6 einstellen. Die Verstellung der Feder ermöglicht somit eine Berücksichtigung des Widerstandes, daß das biologische Probengut dem Durchstechen entgegensetzt. Durch die Betätigung des Auslösers 12 wird die vorher durch Zurückziehen des Schußbolzens 7 gespannte Schußfeder ausgelöst. Der Schußbolzen schlägt nun mit der entsprechend eingestellten Kraft auf eine in der Vorrichtung befindliche Schußnadel, vor die. durch das Spannen des Schußbolzens mittels des Magazins 10 im Griff 11 ein Probendeckel geladen wurde. Durch die kinetische Energie des Schußbolzens wird die Schußnadel mit dem davor befindlichen Probendeckel durch das Probengut getrieben und der Probendeckel mit der darin befindlichen Probe wird in die sich im Probenbehältermagazin 3 befindlichen Probenbehälter gedrückt und bildet somit eine fest verschlossene Probenkapsel im Magazin 3 auf der Gegenhalterseite 9. Eine Rückholfeder an der Schußnadel gewährleistet ein anschließendes sofortiges Zurückziehen der Schußnadel aus dem Probanden, um eine Verletzung durch eine ruckartige Bewegung des Probanden zu vermeiden. Das Behältermagazin 3 wird bei jedem erneuten Spannen der Schußfeder automatisch durch eine Mechanik um eine Behälterposition versetzt. Das Gelenk 13 zwischen dem eigentlichen Gerät und dem Gegenhalter 9 kann um 90 Grad nach unten geschwenkt werden und ermöglicht gegebenenfalls eine bessere Zugänglichkeit der Vorrichtung zum Einlegen des zur Probenentnahme vorgesehenen Gewebes. Einen weiteren wichtigen Aspekt erfüllt die in der Vorrichtung integrierte Elektronik hier zum Beispiel bestchend aus einem alphanumerischen Display 1 z.B. eine LCD-Matrixanzeige, einer numerischen oder alphanumerischen Tastatur 2 zur Dateneingabe und Bestätigung, einem Medium zur Datenspeicherung 5 z.B. eine Chipkarte mit zusätzlichem Magnetstreifen und einem Barcodeleser 4. Die Besonderheit der Vorrichtung bildet hierbei die sinnvolle Kombination von Elektronik und sicherheitsrelevantem Sperrmechanismus der Mechanik um Probenverwechsclungen auszuschließen. Der zum Laden in das Magazin 3 einzulegende Behälterstreifen (Fig. 12) ist durch eine einseitig am Behälterstreifen angebrachte Fahne mit fortlaufender Numerierung durch einen Barcode gekennzeichnet, der mit Hilfe des Barcodelesestiftes 4 vor dem Einlegen gelesen wird. Die Elektronik der Vorrichtung initialisiert die in den Kartenscheiber vorher eingesteckte Chipkarte 5 und speichert die eingelesene Nummer des Barcodes. Der Probenbehälterstreifen (Fig. 12) läßt sich in das Magazin 3 nur in einer Richtung einlegen und in die Vorrichtung laden. Dies läßt nur eine definierte Entnahmereihenfolge zu. Die Elektronik sperrt nach dem Laden der Vorrichtung mit dem Behälterstreifen den Auslöser 12 und fordert über das Display zur Eingabe einer den Probanden eindeutig identifizierende Nummer auf. Diese Nummer kann zum Beispiel die Zuchtregisternummer von Rindern sein. Die Eingabe dieser Nummer erfolgt über die Tastatur.

Zusätzlich wird sie auf dem Display angezeigt. Nach einer Bestätigung auf der Tastatur wird diese auf der Speicherkarte 5 für die erste zu entnehmende Probe abgespeichert und die Elektronik entsichert den Auslöser 12. Das Display I fordert zur Probenentnahme auf. Nachdem die Probenentnahme durch den Bediener vollzogen wurde sperrt die Elektronik den Auslöser 12 und es wird um die Bestätigung einer erfolgreichen Probenentnahme durch das Display aufgefordert. Über die Tastatur wird diese Frage durch Drücken einer bestimmten Taste bestätigt oder durch Drücken einer anderen Taste storniert. Diese Information wird von der Elektronik der Vorrichtung ebenfalls auf der Speicherkarte gespeichert. Nach dem erneuten Zurückziehen des Schußbolzens 7 wird aus dem Dcckelmagazin 10 automatisch ein neuer Probendeckel geladen und das Behältermagazin 3 rückt um ein Probenbehälterposition weiter und der hier beschriebene Vorgang der elektronischen Dateneingabe, bzw, der Freigabe der Vorrichtung beginnt erneut. Die beschriebene Routine wiederholt sich bis alle im Probenbehältermagazinstreifen (Fig. 12) enthaltenen Probenbehälter - hier 16 Stück - gefüllt sind und die Vorrichtung mit neuen Probendeckeln, einem neuen Probenbehälterstreifen sowie einer neuen Chipkarte 5 bestückt werden muß. Durch die eindeutige Zuordnung der Daten auf der Speicherkarte durch den gespeicherten Barcode und dem Barcode des Probenbehälterstreifens können die Probenbehälterstreifen sowie die Chipkarte zusammen oder getrennt zur weiteren Auswertung und Analyse z.B. mit der Post an ein Labor geschickt werden.

Fig. 2 zeigt eine einfache Mechanik in zangenähnlicher Form, die zum Beispiel für geringere Probenentnahmezahlen geeignet wäre und keine direkte Dateneingabemöglichkeit erfordert. Eine mögliche Anwendungen ist die biologische Probenentnahme bei Pflanzen. Die Zange besteht im wesentlichen aus vier Hauptkomponenten. Die Besonderheit liegt in der speziellen Gestaltung der linken und rechten Zangenbacke 16 und 14. Die rechte Zangenbacke besitzt einen speziellen Aufnahmedorn zum Aufstecken des Kapseldeckels. Die linke Zangenbacke 16 ist mit einer Bohrung versehen, welche zur Aufnahme der Probenbehälter 17 bestimmt ist. Die beiden Zangenhälften 21 und 22 bilden gleichzeitig die Zangengriffe und sind durch den gemeinsamen Drchpunkt 18 miteinander verbunden. Eine Feder 19 um den Drehpunkt 18 dient zum Wiederaufspreizen der Zange. Die besondere Formgebung der beiden Zangenhälften sowie deren Verbindung mit den Zangenbacken 16 und 14 ermöglicht ein paralleles Schließen und Öffnen der Zangenbacken. Zur Probenentnahme wird die Zange mit dem Kapseldeckel 15 und dem Probenbehälter 17 bestückt. Wenn sich biologisches Probengut 67 zwischen den beiden Zangenbacken befindet, werden durch Zusammendrücken der Zangenbälften die Zangenbacken parallel geschlossen. Die Probe wird durch den Kapseldeckel 15 und den als Matrize dienenden Probenbehälter 17 aus dem Probengut ausgestanzt. Durch das vollständig Schließen der Zange fügt sich der Probendeckel mit dem Probenbehälter zur fest verschlossenen Probenkapsel mit der darin enthaltenen Probe zusammen. Das Öffnen der Zange bewirkt, daß der Dorn der Zangenbacke 14 sich aus dem Probendeckel herauszieht. Nach dem vollständigen Öffnen der Zange kann die zur Einheit gefügte Probenkapsel aus der Zangenbacke 16 zur weiteren Analyse leicht entnommen werden.

Fig. 3 zeigt eine Probenkapsel, die auf stanzende Art und Weise eine Probe entnehmen kann, indem der Probenbehälter als 22 Matrize und der Probenkapselverschluß 23 als Stempel dient. Der Probenbehälter verfügt üher eine kleine Aussparung 24 am Boden, die als mögliche Einstichstelle für eine Kanüle dient, um so zum Beispiel Flüssigkeiten einzubringen oder zu entnehmen. Der Probenkapselverschluß verfügt auf der unteren Seite über eine scharfe Außenkante 25, die das Abscheren des Gewebes erleichtern soll und über eine Vertiefung 26 eine Mindestprobenmenge garantiert. Durch eine runde Nut 27 im Probenkapselbehälter und dem passenden Gegenstück im Probenkapselverschluß wird ein unerwünschtes Öffnen einer verschlossenen Probenkapsel verhindert.

Fig. 4 zeigt eine Probenkapsel, die auf schießende Art und Weise eine Probe entnehmen kann, indem der Probenkapseldeckel 28 durch die dazugehörige Vorrichtung durch das Probenmaterial bindurch geschossen und vom Probenbehälter 29 aufgefangen wird. Der Probenkapseldeckel 28 verfügt über eine konisch zulaufende Spitze, die den Widerstand beim Durchdringen des Materials senkt und das Einführen in den Probenbehälter erleichtert sowie über eine zylindrische Vertiefung 30, in der die Gewebeprobe gesammelt wird. Durch eine scharfkantige Nut 31 im Probenbehälter und im Probenkapseldeckel ergibt sich die Funktion eines Schnappverschlußes, um ein nicht nachweisbares unrechtmäßiges Öffnen einer verschlossenen Probenkapsel zu verhindern.

Fig. 5 zeigt eine Probenkapsel, die auf stoßende (geführtes Schießen) Art und Weise eine Probe entnehmen kann, indem der Probenkapseldeckel 32 durch die Vorrichtung mittels einer Führung, welche durch den Ausatz 33 und der Vertiefung 34 am hinteren Ende der Probenkapsel mit der Führung verbunden ist, durch das Probenmaterial gestoßen wird. Die Vertiefung 34 kann auch als Einslichstelle für Kanülen dienen und der Ansatz 33 zum automatisierten Entfernen des Probenkapseldeckels. Die Probenbehälter 35 verfügt über ein Septum 36, welches zum Beispiel aus einem eingelassenem Stück Gummi besteht.

Fig. 6 zeigt eine Probenkapsel, die eine Gewebeprobe entnehmen kann, indem der Probenkapseldeckel 37 mit der Spitze 38, bevorzugt unter Verwendung einer Vorrichtung, in den Probanden kurz und ruckartig hineingestochen und direkt wieder herausgezogen wird. Dabei setzen sich kleine Mengen des zu untersuchenden Gewebes am Widerhaken 39 fest und der Probenkapseldeckel einschließlich der Gewebeprobe kann in den Probenhehälter 40 eingeführt und verschlossen werden.

Fig. 7 zeigt eine Probenkapsel, die auf kneifende bzw. knipsende Art und Weise eine Probe entnehmen kann. Dies geschieht, indem der Prabenkapseldeckel 41 durch die dazugehörige Vorrichtung, welche den Deckel an dem Ansatz 42 fixiert, eine radiale Bewegung vom geöffneten zum geschlossenen Zustand ausführt. In dem Moment, in dem die an der vorderen Seite scharfe, spitz zulaufende Nase 43 am Öffnungsrand des Probenbehälters 44 vorbei geführt wird eine Gewebeprobe abgekniffen.

Fig. 8 zeigt eine Probenkapsel, die wie Figur 7 auf kneifende bzw. knipsende Art und Weise eine Probe entnehmen kann, bei der aber bei Bedarf auf eine zusätzliche Vorrichtung verzichtet werden kann. Der Probenbehälter 45 und der dazugehörende Deckel 46 bestehen aus einem Teil und sind über das Gelenk 47 miteinander verbunden. Die radiale Bewegung geschieht um das Gelenk und kann von Hand ausgeführt werden.

Fig. 9 zeigt eine Probenkapsel die Ähnlichkeiten zu einer Biopsienadel aufweist. Der probenehmende Teil 48, der auf einer Seite verschlossen ist und auf der anderen Seite eine schneidende, schräge Kante 49 besitzt, kann entweder unter Benutzung der entsprechenden Vorrichtung durch das zu untersuchende Material hindurchgeführt oder in den Probanden hineingestochen und wieder herausgezogen werden. Nach der Probenentnahme wird der probenehmende Teil 48 in einen Behälter 50 eingeführt oder mit einem Deckel 51 verschlossen.

Fig. 10 zeigt eine Probenkapsel, die auf kratzende oder schabende Art und Weise eine Probe entnehmen kann, indem der Prohenkanscleteckel 52 durch die dazugehörige Vorrichtung oder manuell an einer geeigneten Oberfläche des Probanden unter einem gewissen Druck entlang geriehen wird. Gewebeteile werden dabei von den Lamellen oder Zähnen 54 entnommen und gespeichert. Der mit Probenmaterial bestückte Probenkapseldeckel 52 kann dann bis zum Anschlag 55 in den Probenbehälter 53 eingeführt und somit fest verschlossen werden.

Fig. 11 zeigt eine Probenkapsel, die ähnlich wie Figur 10 auf kratzende Art und Weise eine Probe entnimmt aber mit dem Unterschied, daß der Probendeckel 56 als Prohennchmer mit den Kratzern 58 in einer radialen Bewegung am Probanden vorbeigeführt und in den Probenbehälter 57 gesteckt wird.

Fig. 12 zeigt gegurtete Behälterstreifen mit angebrachter Fahne und Barcode.

Fig. 13 zeigt eine andere mögliche Zusammenfassung der Probendeckel in rotationssymetrischer

Form für den Fall das keine rechteckigen Schaftmagazine sondern Trommelmagazine verwendet werden.

Fig. 14 zeigt die Gestaltung von Probenbehälter und Probendeckel in Kombination mit einer bei der Probenentnahme automatisch angebrachten Kennzeichnungsmarke z.B. mit einem in der Marke integriertem codierten Transpondersender. Dazu ein im Spritzgußverfahren hergestellter Probenbehälter 60 an dem mit kleinen Stegen 61 verbunden eine Scheibe 59, ähnlich einer Zahnscheibe mit Innenverzahnung 62, hängt, die den Zweck eines Schießkopfes erfüllt.

Fig. 15 zeigt das zugehörige Gegenstück zu Fig. 14 mit Transpondersender 66 und einem hohlnietförmigen Ansatz. Der zylindrische Teil des Ansatzes besitzt einen Einstich in Form einer rotationssymetrischen Nut 65, in die die Verzahnung 62 des Schließkopfes einrastet und somit einen Verbund bildet. Der Kapseldeckel 64 wird bei der Herstellung separat gefertigt und anschießend in den Hohlteil auf der Scheibenseite bündig eingesteckt, so daß beide Teile eine Einheit bilden. Eine zweckmäßige Anwendungsmöglichkeit für diese Kombination aus Probenkapselteilen und zur Markierung geeigneten Teilen findet sich zum Beispiel bei einer massenhaften Probenentnahme zur Typisierung von Rindern durch einen genetischen Fingerprint. Die bei der Probenentnahme automatisch angebrachte Marke (Fig. 14, Fig.15) mit Identifikationsnummer durch ein Transpondersender 66 ermöglicht die eindeutige Zuordnung zwischen dem Rind und der Probe. Weiterhin könnte die Marke herkömmliche Ohrmarken bei Rindern ersetzen, zumal die Identifikationsnummer des Transpondersender berührungslos ausgelesen werden kann.

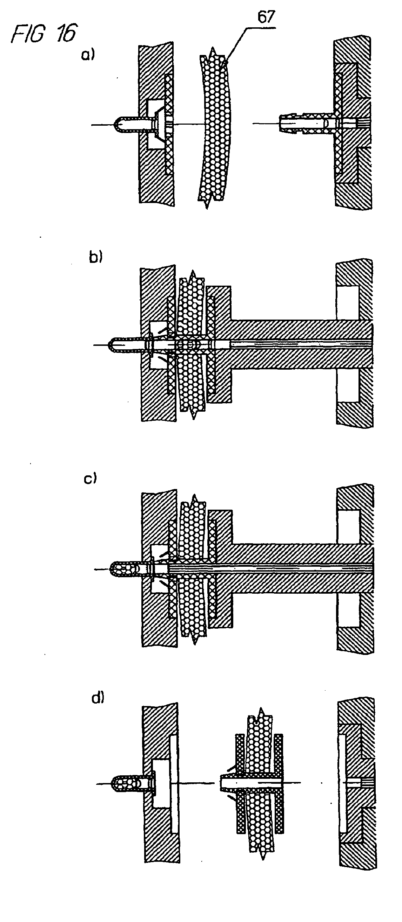

Fig. 16 zeigt im Funktionsprinzip, wie mit einer mechanisch vollautomatischen Vorrichtung wie sie in (Fig. 1) oder in einer einfachen mechanischen Vorrichtung wie sie in (Fig. 2) dargestellt ist zum Einsatz kommen könnte. Fig. 16a zeigt die in der Vorrichtung positionierten Teile mit dem dazwischenliegenden Gewebe zur Probenentnahme. Fig. 16b zeigt wie die Fig. 15 mit einem Stempel durch das Gewebe gestochen wird und im Gegenhufter in die als Nictkopf fungierende Fig. 14 einrastet und im gleichen Schritt den eigentlichen Probenbehälter 60 von der Scheibe 59 abtrennt und im Gegenhalter fixiert. Durch das Durchstechen des Probanden befindet sich Gewebe in dem hohlen Teil der Fig. 15. In Fig. 16c wird das Gewebe im hohlen Teil mittels eines kleineren Dorns, der durch den Stempel geführte wird, von der Vorrichtung weiter heraus geführt und schiebt den sich in Fig. 15 befindlichen Prohendeckel 64 durch den hohlen Teil der Marke (Fig. 15) und befördert somit den Gewebeanteil in den Probenbehälter 60, verschließt diese zugleich mit dem Probendeckel 64 zu einer festen Einheit. Fig. 16d zeigt den von der Vorrichtung wieder zurückgefahrenen Stempel und Dorn, sowie die angebrachte Markierung mit einer möglichen Kodierung 66 und die verschlossene Probenkapsel mit der darin enthaltenen Gewebeprobe.

Fig. 17 zeigt einen zur Probennahme geeigneten Probenkapseldeckel, dessen Besonderheit in einem tief ausgearbeiteten Schlitz 68 liegt, wodurch der vordere Teil des Probennehmers aus zwei spitz zulaufenden Halbrunden 69 besteht. Das untere Ende des Schlitzes ist als Bohrung 70 ausgearbeitet. Zur sicheren Fixierung im Probenbehälter besitzt der Probendeckel eine Nut 71, welche als Vertiefung ausgearbeitet ist, damit das zu untersuchende Gewebe nicht unnötig beschädigt wird.

Fig. 18 zeigt einen Probenkapseldeckel, der in seiner Funktion der Fig. 17 entspricht mit dem Unterschied daß diese Ausführung über zwei stumpf endende Schlitze 72 verfügt so das vier spitz zulaufenden Viertel entstehen.

Fig. 19 zeigt eine weitere Ausführungsvariante der Fig. 17. Hier kommen drei Schlitze 73 zum Einsatz die jeweils trapezförmig 74 ausgearbeitet sind, so daß sechs spitz zulaufenden Teile entstehen.

Fig. 20 zeigt die Fig. 17 nach der Probenentnahme mit dem entnommenen Gewebe 77 und nachdem der Probennehmer 75 als Probenkapseldeckel in den Probenbehälter 67 eingeführt wurde.

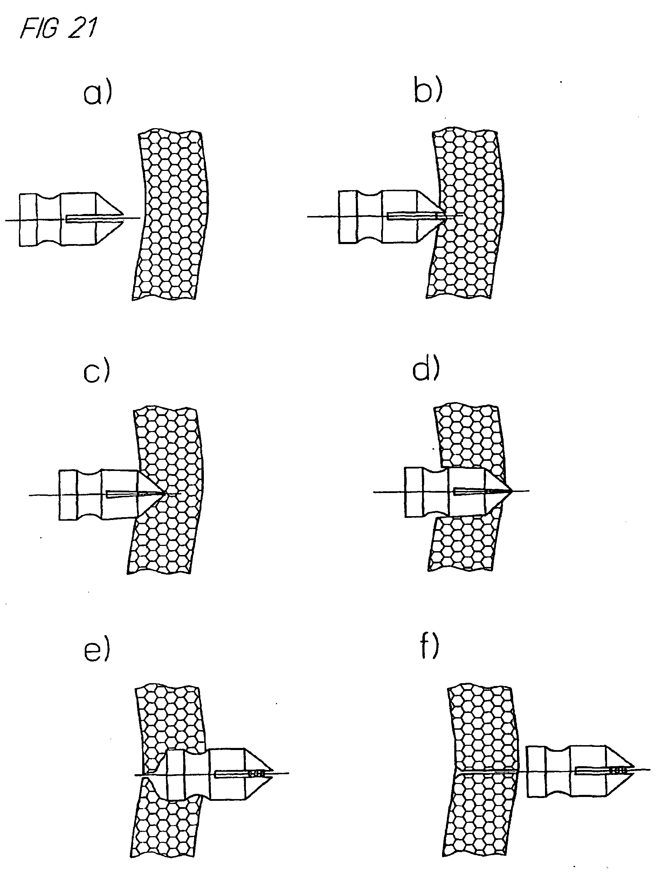

Fig. 21 zeigt in den einzeinen Schritten a) bis f) die Funktionsweise der Probennehmer aus den Figuren 17 bis 19. In a) ist der Probennehmer kurz vor der Berührung mit dem Probenmaterial zu sehen. Es ist vorstellbar, daß der Probennehmer entweder in irgend einer Form beschleunigt wurde und nun kinetische Kräfte besitzt, oder z.B. von einer Führungsstange von hinten gleichmäßig geschoben wird. In b) ist zu sehen, wie die Spitzen des Probennehmers in das Material hineingestochen werden und so eine bestimmte Materialmenge zwischen sich bringen. In c) geschieht die eigentliche Probenentnahme. Die beiden Hälften des Probennehmers werden vom Probenmaterial durch die beiden Schrägen zusammengedrückt und quetschen nun das zwischen ihnen liegende Gewebe ab. In d) ist zu sehen, daß der Probennehmer durch die zusammengedrückten Spitzen das Gewebe durchwandert ohne weiteres Gewebe aufnehmen zu können, In e) und f) ist zu schen, daß der Probennehmer durch den fehlenden Druck von vorne sich wieder geöffnet hat und die entnommene Probe freigibt. Vorteile dieser Methode der Probenentnahme liegen darin, daß der exakte Ort der Probenentnahme auch beim Durchdringen des gesamten Gewebes exakt definiert ist und daß die entnommene Menge der Probe immer konstant gehalten werden kann.

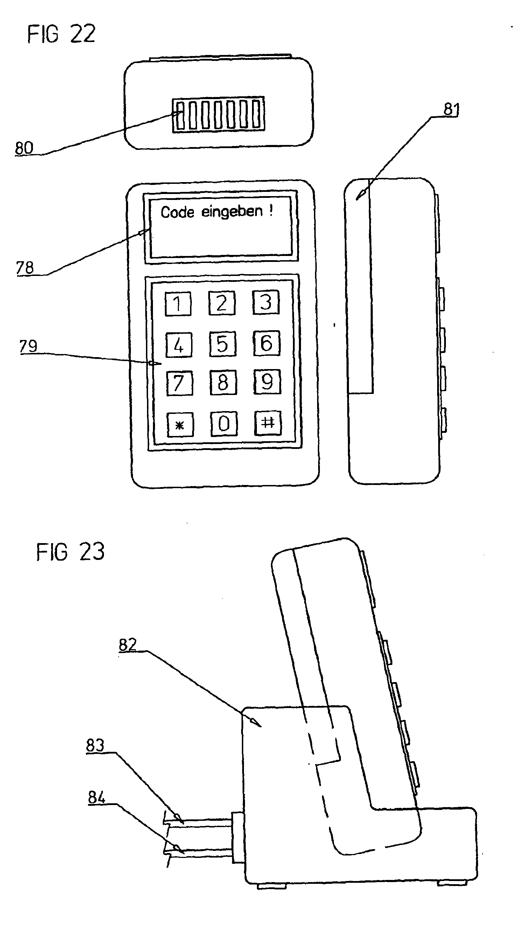

Fig. 22 zeigt ein tragbares Dateneingabegerät mit internem Datenspeicher. Dieses Gerät kann zum Beispiel als Zusatzmodul zu einer Probenentnahmezange wie in Figur 2 dargestellt verwendet werden und ergibt so eine voll kompatible Einheit zu komplexeren Entnahmeeinheiten, wie sie in Fig. 1 dargestellt ist. Die numerische oder alphanumerische Tastatur 79 ermöglicht dem Benutzer die Eingabe von Daten z.B. die Zuchtregisternummer bei Rindern oder andere probenspezifische Daten. Das LCD-Display 78 erlaubt dem Benutzer die Kontrolle der Dateneingabe, sowie deren Bestätigung über die Tastatur 79. Weiterhin führt das LCD-Display den Benutzer durch die im Dateneingabegerät programmierte Prozedur zur Abfrage und zur Dateneingabe. Die Batterieeinheit 81 ist für die Stromversorgung des Dateneingabegerätes verantwortlich und kann wahlweise mit Batterien oder Akkumulatoren bestückt werden. Die Ausgabe der intern gespeicherten Daten erfolgt über eine Schnittstelle 80 durch Einstecken in die Basisstation. Zusätzlich kann ein elektronischer Adapter an die Schnittstelle gekoppelt werden, der es ermöglicht das Dateneingabegerät zur Datenausgabe an ein Funktelefon anzuschließen.

Fig. 23 zeigt die Basisstation, welche mit Netzstrom über das Kabel 83 betrieben wird. Ein in die Basisstation eingebautes Modem wird über das Telefonkabel 84 mit einer Teletonsteckdose verbunden und ermöglicht die Datenübertragung an eine andere Datenbank. Das integrierte Ladegerät ermöglicht bei der Verwendung von Akkumulatoren im Dateneingabegerät deren Aufladung. Das Modem in der Basisstation erlaubt weiterhin das Einspielen neuer Dateneingabeprozeduren für benutzerspezifische Anwendungen in den Speicher des Dateneingabegerätes sowie die Überprüfung des Gerätes.

Fig. 24 zeigt eine Grafik zur Verdeutlichung des Identitätsnachweises zwischen Probe des Probanden (z.B. vom Rind) und entnommener Probe des Fleisches (z.B. vom Schlachthof oder dem Verkauf). Die Probe des Probanden, die sich im Probenbehälter befindet wurde mit der Vorrichtung entnommen. Der Proband erhält von der Vorrichtung eine Markierung mit Kodierung. Die Daten über die spezifischen Daten des Probanden sowie Kodierung der Markierung und die Position der Probenbehälter auf dem Probenstreifen wird von der Vorrichtung auf einem Speichermedium gespeichert und mit der Post zur Datenbank geschickt oder direkt mittels Modem über Telefon bzw. Funktelefon an eine Datenbank übergeben. Der Probestreifen wird mit der Post zur Analyse geschickt dort analysiert und die Daten der Analyseergebnisse zur Datenbank transferiert. Die entnommene Fleischprohe durchläuft die gleiche Prozedur. Die Datenbank ermöglicht nun den eindeutigen Identitätsnachweis ob ein Fleischprodukt wahrheitsgemäß seiner Deklaration mit dem Rind übereinstimmt oder nicht.

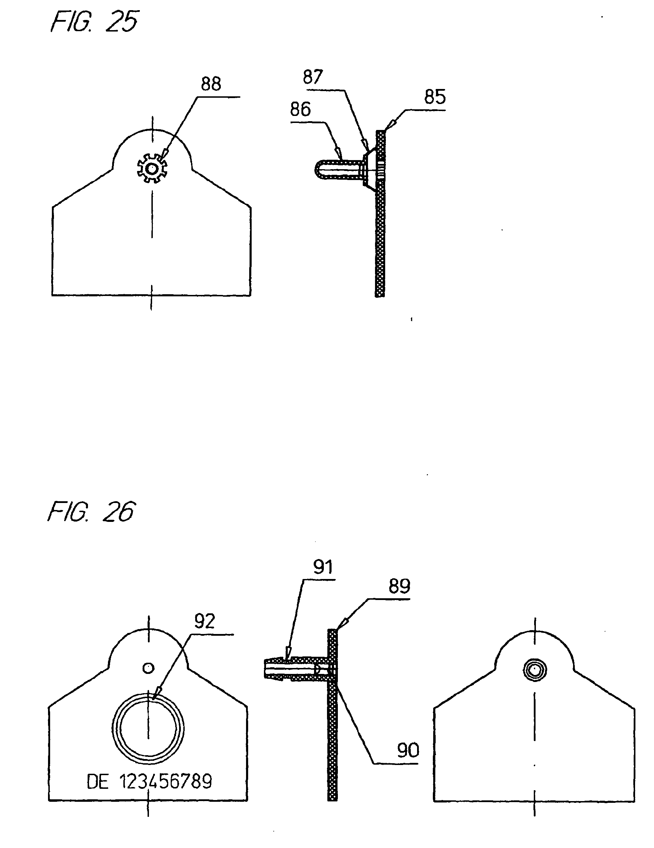

Fig 25 zeigt ein weiteres Beispiel für die Gestaltung von Prohenbehälter und Probendeckel in Kombination mit einer bei der Probenentnahme automatisch angebrachten Kennzeichnungsmarke wie sie zur Zeit bei Rindern benutzt werden mit einem in der Marke integriertem codierten Transpondersender. Der z.B. im Spritzgußverfahren hergestellte Probenbehälter 86 ist mit kleinen Stegen 87 an der Scheibe 85 befestigt. Die Scheibe 85 besitzt eine Innenverzahnung 88 ähnlich einer Zahnscheibe, die den Zweck eines Schießkopfes erfüllt.

Fig. 26 zeigt das zugebörige Gegenstück zu Fig. 25 mit Transpondersender 92 und einem hohlnietförmigen Ansatz. Der zylindrische Teil des Ansatzes besitzt einen Einstich in Form einer rotationssymetrischen Nut 91, in die die Verzahnung 88 des Schließkopfes einrastet und somit einen Verbund bildet. Der Kapseldeckel 90 wird bei der Herstellung separat gefertigt und anschießend in den Hohlteil auf der Scheibenseite bündig eingesteckt, so daß beide Teile eine Einheit bilden. Eine zweckmäßige Anwendungsmüglichkeit für diese Kombination aus Probenkapselteilen und zur Markierung geeigneten Teilen findet sich zum Beispiel bei einer massenhaften Probenentnahme zur Typisierung von Rindern durch einen genetischen Fingerprint und gleichzeitiger Kennzeichnung mittels Ohrmarke und der entsprechenden Eintragung im Zuchtregister. Die bei der Probenentnahme automatisch angebrachte Marke (Fig. 25, Fig.26) mit Identifikationsnummer durch ein Transpondersender 92 ermöglicht die eindeutige Zuordnung zwischen dem Rind und der Probe. Weiterhin könnte die Marke herkömmliche Ohrmarken bei Rindern ersetzen, zumal die Identifikationsnummer des Transpondersender berührungslos ausgelesen werden kann.

Das mögliche Funktionsprinzip für diese Probenkapsel (Fig.25, Fig 26) ist identisch mit dem in Fig. 16 bereits gezeigten und beschriebenen, wie es zum Beispiel in einer mechanisch vollautomatischen Vorrichtung, wie sie in (Fig. 1) oder in einer einfachen mechanischen Vorrichtung wie sie in (Fig. 2) dargestellt ist, zum Einsatz kommen könnte.

### Verwendungsbeispiele

Eine der aktuellsten Anwendungen ist der erste Schritt in einem Verfahren, welches von der Firma Genom Analytik GmbH (GAG) Bremen, zur Registrierung und Kontrolle aller Nutztiere entwickelt wird. Die Vorrichtung ist ein wichtiger Bestandteil des Verfahrens. Ein Flußschema des Verfahrens ist in Figur 24 dargestellt. Die Rolle der erfindungsgemäßen Vorrichtung geht aus dieser hervor.

Ein Zuchtverband oder andere mit der Herkunftskontrolle beauftragte Organisation bestellt beim Hersteller der erfindungsgemäßen Probenkapsel eine bestimmte Menge von Probenkapseln, welche auf eindeutige Weise mit Seriennummern markiert geliefert werden. Der Hersteller der Probenbehälter speichert elektronisch die Seriennummern und an wen welche Probenbehälter geliefert wurden. Eine Seriennummer kann dabei zum Beipiel 10-stellig sein, was es möglich macht bis zu 80 Milliarden Probenbehälter herzustellen, ohne daß jemals eine Seriennummer zweimal im Umlauf ist (jede Seriennummer kennzeichnet einen Streifen von mindestens 8 Behältern). Die kontrollierende Organisation bezieht für sich selber oder die einzelnen Landwirte oder Milchkontrolleure (dies gilt für Deutschland, in anderen Länder kontrollieren andere Organisationen) eine Anzahl Probenentnahmevorrichtungen.

Mit diesen Vorrichtungen ausgestattet besucht die kontrollierende Person die Landwirte einer Region, welche per Gesetz dazu verpflichtet sind, alle Geburten von Rindern zu melden. Der Zuchtverband vergibt für jeden Landwirt, welcher Geburten gemeldet hat die entsprechenden Herdbuchnummern, welche der probenentnehmenden Person üherreicht werden. Die mit den Behältern, der Vorrichtung und den Herdbuchnummern ausgestatteten Kontrolleure führen auf jedem Gut den folgenden Prozeß durch. Die Vorrichtung wird mit den Probenbehältern und Probenkapseldeckeln geladen. Dabei kann jeder Probenbehälter aus zwei Komponenten bestehen, dem eigentlichen Behälter und einem Teil einer Ohrmarke. In diesem Fall besteht auch der Deckel der Probenkapsel aus zwei Teilen, dem eigentlichen Deckel, weicher gleichzeitig die Probenentnahme vollzicht und einem weiteren Teil der Ohrmarke. In einer bevorzugten Verfahrenvariante werden dabei die Probenbehälter in Form von Streifen oder Ringen geliefert, an denen sich mehrere solcher Behälter befinden. Diese Behälter sind so zu einem Streifen verkettet, daß sie nicht ohne die offensichtliche Beschädigung des Streifen voneinander getrennt werden können. Die Deckel können entweder einzeln, in Streifen oder Ringen geliefert werden.

Da diese bei der Probenentnahme vom Streifen oder Ring getrennt werden, ist es nicht notwendig diese fest miteinander verschweißt zu liefern. Werden die Deckel als Streifen oder Ring geliefert, so geschieht dies hauptsächlich zwecks einfacherer Handhabung der Vorrichtung während des Ladevorgangs. Einer der beiden Teile der Ohnnarke kann entweder mit einer Seriennummer (zum Beispiel direkt der Herdbuchnummer) versehen sein, oder eine solche Nummer in Form eines Senders oder Transponders tragen. Wird nun die erfindungsgemäße Vorrichtung mit den erfindungsgemäßen Probenbehältern und Probenkapseldeckeln mit Ohrmarke geladen geschicht das folgende: Die Vorrichtung liest automatisch die Seriennummer. Figur 12 zeigt eine mögliche Variante des Behälterstreifens, bei welcher ein Barcode in Form einer "Fahne" in der Art am Streifen befestigt, daß die Laderichtung vorgegeben ist. Durch eine Nummer sind alle weiteren Behälter auf einem Streifen eindeutig gekennzeichnet. Darauf hin fordert die Vorrichtung die Eingabe einer genauen Bezeichung des Lebewesens. Die Vorrichtung kann dabei so programmiert sein, daß eine nicht der richtigen Form entsprechende Eingabe sofort in einer Fehlermeldung resultiert. In diesem Fall kann keine Probenentnahme stattfinden, die Vorrichtung wird nicht freigegeben. Die Vorrichtung kann außerdem durch weitere Programme einen Plausibilitätstest der Eingabe vornehmen. Zum Beispiel könnten bestimmte Exemplare der Vorrichtung, welche nur lokal in bestimmten Regionen verwendet werden, nur bestimmte, nur in diesen Regionen verwendete Codes entgegennehmen. Zum Beipiel kann programmiert werden, daß eine in Deutschland ausgelieferte Vorrichtung nur Herdbuchnummern mit der Kennung "DE" für die Freigabe der Probenentnahme entgegennimmt.

Wenn die Probenbehälter und Deckel mit den Teilen einer Ohrmarke gemeinsam angeliefert werden, muß die Vorrichtung in der Lage sein, jeder Ohrmarke eine eindeutige Kennung zuzuordenen. Ein bevorzugtes Ausführungsbeispiel wäre, daß ein Teil der Ohrmarke mit einem Transponder versehen ist. Dieser trägt die Information für genau eine Nummer, welche der Nummer des Behälters zugeordnet wird. Die Vorrichtung kann diese information lesen und führt automatisch die Zuordnung der Nummer der Ohrmarke mit der Seriennummer und der Position des Probenbehälters durch, welcher gerade geladen ist. Diese Assoziation kann dabei für die mechanische Freigabe der Vorrichtung zur Probenentnahme verbunden sein. So ist es ausgeschlossen, daß eine Entnahme ohne gleichzeitige Befestigung der Ohrmarke stattfindet.

Die beschriebenen Verfahrensschritte führen zu der folgenden Situtation. In der Vorrichtung sind Informationen zusammengeführt worden über die genaue Kennzeichnung des Probenbehälters, über die an dem Tier verbleibende Nummer und die zum Beispiel vom Zuchtverband vergebene Herdbuchnummer. Diese Informationen werden untrennbar von der Vorrichtung gespeichert. Erst wenn alle diese Schritte vollzogen sind, signalisiert die Vorrichtung, daß nun eine Probenentnahme stattfinden kann. Dies kann durch ein Lichtzeichen, ein akustisches oder äquivalentes Signal erfolgen.

Die eigentliche Probenentnahme wird in der bevorzugten Vorrichtungsvariante (Fig. 1) mechanisch ausgeführt. In verschiedenen Varianten kann die Vorrichtung vor der Entnahme gespannt und dann durch einen Auslöser ausgelöst werden oder wie ein Tacker mechanisch betätigt werden oder mit einem Motor die eigentliche Entnahme antreiben. In einer Verfahrensvariante wird ein Probenstreifen bis zu einem definierten Punkt in die Vorrichtung eingeführt und dünn nach jeder Entnahme automatisch ein neuer Deckel (mit Marke) geladen und der Bchälterstreifen um eine Position vorgeschoben, so daß der Deckel sich in einer Linie mit einem Stanzbolzen befindet. Die mechanische Auslösung hat also zwei Folgen. Der Stanzbolzen wird in die Vertiefung des Deckels eingeführt und unter Mitnahme einer Gewebeprobe durch das Gewebe in den Behälter getrieben und anschließend nach dem rückwärtigen Bewegungsablauf des Stanzbolzens mit beiden Teilen einer Probenkapsel neu geladen. Die Vorrichtung wird für weitere Probenentnahmen blockiert, bis ein kompletter neuer Satz Informationen eingegeben ist.

Das Einstanzen des Deckel in den Probenbehälter hat zur Folge, daß dieser so verschlossen ist, daß kein Öffnen mehr möglich ist, ohne daß der Behälter sichtbar beschädigt wird. Damit ist sichergestellt, daß die sich im Behälter befindliche Gewebeprobe untrennbar mit der Herdbuchnummer und Ohrmarkennummer assoziiert ist. Die Möglichkeit von Mißbrauch wird dadurch erheblich eingeschränkt. Teil des weiteren Verfahren ist es, daß Probenkapselstreifen (mit Gewebe) auf Beschädigung untersucht werden. Es ist dem Hersteller (und damit der Analysestelle) bekannt, in welchen Einheiten die Behälter ausgeliefert werden. Ein Abtrennen einzelner Behälter von einem Streifen wird offensichtlich. Nach dem Befüllen eines kompletten Streifens von Behältern wird dieser aus der Vorrichtung entnommen und kann verschickt werden. Die Streifen sind dabei extrem robust aber flexibel. Die Kapseln sind wasserdicht verschlossen, so daß auch ein versehentliches Fallenlassen in zum Beispiel eine Wasserlache keinen Einfluß auf die Probe hat.

Ein (in Deutschland) Milchkontrolleur füllt auf diese Art und Weise über einen bestimmten Zeitraum eine Anzahl Probenkapselstreifen. In regelmäßigen Abständen, werden die gesammelten Proben verpackt und versandt.

Die Weitergabe der Daten, welche in der Vorrichtung gespeichert sind kann, je nach Organisationsstruktur der Region, des Landes oder Verbundes auf verschiedene Weise erfolgen. Ein bevorzugtes Beipiel wäre die Speicherung der Daten auf einer Chipkarte. Auf dieser Karte befindet sich ein lesbarer und beschreibbarer elektronischer Chip, welcher alle Informationen über eine bestimmte Anzahl von Proben entgegennehmen kann. Ist die Kapazität des Chips erschöpft, so blockiert die Vorrichtung jede weitere Probenentnahme. Eine Chipkarte kann, nachdem der Chip vollständig beschrieben wurde oder eine minimale Anzahl von Entnahmen auf dem Chip dokumentiert wurden, aus der Vorrichtung entnommen werden und separat oder zusammen mit den gefüllten Probenkapselstreifen versandt werden. Zusätzlich kann die Seriennummer der Vorrichtung, Informationen über die entnehmende Person und die genauen Kalenderdaten der Entnahmen auf dem Chip gespeichert werden. Der Chip kann außerdem mit einer Geheimmummer so blockiert werden, daß dieser nur von einer eingetragenen Vorrichtung bei Verwendung von registrierten Behälternummern beschrieben werden kann. Außerdem könnte jedes Entnehmen der Karte aus einer Vorrichtung jedes weitere Beschreiben endgültig bis zu einer erneuten Freigabe durch die zentrale Analysestelle blockieren. Dadurch entsteht ein weiterer Kontrollmechanismus.

Statt der Verwendung einer versendbaren Chipkarte kann auch eine der folgenden Verfahrensvarianten angewendet werde. Die Vorrichtung kann über eine Schnittstelle zu einem Computer verfügen, über welche die dokumentierten Daten über Probenenlnahmen übertragen werden. Die Vorrichtung könnte über einen Kodieralgorithmus verfügen, welcher alle Daten direkt in der Art speichert, daß sie nur mittels eines entsprechenden Kodes, welcher nur der zentralen Analysestelle bekannt ist, zu interpretieren ist. Dadurch ist jedes manipulieren der gespeicherten Informationen unmöglich, da deren Inhalt nicht zu entziffern ist. Gleichzeitig können mit den Informationen über die Proben, Marken, Herdbuchnummern und Lebewesen, selbst Informationen über Datum, Probenentnehmer und weitere Sicherheitskodes gespeichert werden. Eine solche Vorrichtung erlaubt dem Benutzer keinen wie auch immer gearteten Zugriff auf die eingegebenen und die in der Vorrichtung generierten Daten. Vom Computer können alle Daten über eine Modemverbindung zu einer Empfangsstation gesandt werden. Es ist genauso möglich, die Vorrichtung selbst direkt oder über eine entsprechende Kabelverbindung mit einem Telefonnetz zu verbinden. Dann kann über die numerischen Eingabefunktionen der Vorrichtung eine beliebige Empfangsstation angewählt werden. Dies kann auch direkt durch Anschluß der Vorrichtung an ein Mobilfunknetz erfolgen. Auf diese Art und Weise könnten alle Daten direkt und jederzeit versandt werden.

Ein Datentransfer über eine Internet-Verbindung ermöglicht es von sehr vielen Orten weltweit und kostengünstig alle Daten oder nur Daten über die verwendeten Probenbehälter-Scriennummern an eine zentrale Stelle zu übermitteln, welche für das gesamte System eine Plausibilitätsprüfung durchführen kann. Eine Plausibilitätsprüfung auf diese Weise würde beinhalten, daß jede Vorrichtung in regelmäßigen Abständen die Seriennummern der verwendeten Probenbehälter versendet. Es kann dann überprüft werden, ob Seriennummer doppelt vorkommen oder an Orten verwendet werden, für welche diese nicht vorgesehen sind. Da nur eine zentrale Stelle (zum Beispiel der Hersteller) über die Information verfügt, welche Seriennummern produziert worden sind und in welcher Reihe diese in Zukunft produziert werden (kann nicht-durchlaufend, nach einem geheimen System geordnet erfolgen) besteht ein ausgedehnter Schutz des Verfahrens gegen Manipulation oder Verfälschung der Behälter. Damit ist eine Möglichkeit eingeschränkt, durch welche die Fälschungssicherheit des Systems durchbrochen werden könnte.

### Beispiel zur Registrierung von Rindern

Die beschriebene Vorrichtung zur Entnahme von biologischen Proben wird vorbereitet indem die Daten des Rindes, dessen Probe genommen werden soll, über das Tastenfeld in die Vorrichtung eingegeben werden. Dadurch wird die Vorrichtung zur Probenentnahme freigegeben. Aus dem Kuhohr wird die Gewebeprobe durch Schiessen mit der Vorrichtung unter gleichzeitiger Hinterlassung einer Marke entnommen. Auf der Marke befindet sich ein Datenspeicher (Zahlen und Buchstabenbencode, Barcode, Transponder oder Chipkarte). Diese Daten werden den eingegebenen Daten zugeführt. Nach der Probenentnahme von mehreren verschiedenen Rindern wird der Gurt mit den Probenkapseln aus der Vorrichtung genommen und zum Analysenort geschickt. Dort wird eine Genotypisierung an der DNA Probe vorgenommen. Dies kann mittels Massenspektrometric oder konventionellen Methoden durchgeführt werden. Der genetische Fingerabdruck wird dem Herdbuch zugeführt.

### Beispiel zur Registrierung von Pferden

Die beschriebene Vorrichtung zur Entnahme von biologischen Proben wird vorbereitet indem die Daten des Pferdes, dessen Probe genommen werden soll, über das Tastenfeld in die Vorrichtung eingegeben werden. Dadurch wird die Vorrichtung zur Probenentnahme freigegeben. Mit der Vorrichtung werden einige Haare ausgerissen (durch ein sanftes Bürsten des Pferdes). Die Vorrichtung überträgt die Haarwurzeln in die Probenkapsel. Nach der Probenentnahme von mehreren verschiedenen Pferden wird der Gurt mit den Probenkapseln aus der Vorrichtung genommen und zum Analysenort gebracht. Dort wird eine Genotypisierung an der DNA Probe vorgenommen.

### Beispiel zur Registrierung von Schweinen

Die beschriebene Vorrichtung zur Entnahme von biologischen Proben wird vorbereitet indem die Daten der Schweine, deren Proben genommen werden sollen, auf ein offizielles Formular mit einem korrespondierenden Code (Barcode) zu dem auf dem Probenkapselgurt eingetragen werden. Dadurch wird die Vorrichtung zur Probenentnahme freigegeben. Durch Stanzen wird die Gewebeprobe mit der Vorrichtung unter gleichzeitiger Hinterlassung einer Marke entnommen. Auf der Marke befindet sich ein Datenspeicher (Zahlen und Buchstabenbencode, Barcode, Transponder oder Chipkarte). Nach der Probenentnahme von mehreren verschiedenen Schweinen wird der Gurt mit den Prohenkapseln aus der Vorrichtung genommen und zum Analysenort gebracht. Dort wird eine Genotypisierung an der DNA Probe vorgenommen.

### Beispiel zur Gewebeentnahme beim Menschen

Vor der Exeission einer malignen Wucherung nimmt der Chirurg mit der beschriebenen Vorrichtung Gewebeproben in der Wucherung und im benachbarten Gewebe. Die Vorrichtung für diesen Fall ist so konstruiert, daß Gewebe mit einem Kneifmechanismus erfaßt und abgekniffen wird. Das Kneifwerkzeug wird zur Probenkapsel verschlossen. Es kann sinnvoll sein Reagenzien zur Weiterverarbeitung in einem Teil der Probenkapsel vorzulegen (z.B. Proteinase K um die intakten Zellen aufzubrechen und Proteine zu verdauen). Gleichzeitig mit der Gewebeentnahme wird durch die Vorrichtung eine Zahl an den Ort der jeweiligen Probenentnahme gesprüht (Tintenstrahl) oder eine andere Markierung am Probenentnahmort hinterlassen. Die in den gegurteten Probenkapseln befindlichen Proben werden einer Schnellanalyse z.B. Genotypisierung mittels PCR in einem Mikrosystem und einem Flugzeitmassenspektrometer zugeführt. Innerhalb von 15 Minuten erhält der Chirurg seine Analysenresultate und kann nun entscheiden, wieviel Gewebe um den Tumor herum bereits von Krebszellen befallen ist. Es ist dadurch erstmals möglich den plastischen Schaden einer Exeission so klein wie müglich zu halten.

### Ausführungsbeispiel auf dem Gebiet der markerunterstützten Pflanzenzüchtung

Eine Kreuzung einer Wildsorte mit einer Nutzpflanze hat den Zweck die Gene für eine gewünschte Eigenschaft z.B. eine Schädlingsresistenz, welche die Wildsorte aufweist, auf die Nutzpflanze zu übertragen. Es ist bekannt, daß die Resistenz durch mehrere verschiedene Gene der Wildsorte übertragen wird. Bekannt sind meist nur genetische Marker, die auf den Chromosomen sehr nahe bei den entsprechenden Genen liegen, nicht aber die Gene selber oder deren genauer Ort im Genom der Pflanze. Diese genetischen Marker liegen in mehreren Allelen vor (molekular unterscheidbare Versionen derselben Marker in verschiedenen Individuen) vor. Es ist bekannt, welche Versionen der an die Gene "gekoppelten" Marker in der Wildpflanze und der Nutzpflanze vorliegen. Einige hundert verschiedene Marker der Wild - und der Nutzpflanze sind bekannt und können für die Züchtung genutzt werden..

Eine Kreuzung der Wildpflanze mit der Nutzpflanze erzeugt eine Population von Tausenden von Pflanzen, von denen nur wenige die erwünschte Kombination der Marker und damit der Gene enthalten. Außerdem haben diese Pflanzen individuell verschiedene prozentuale Anteile an Genen der Wildpflanze. Die Wildpflanze trägt oft eine Reihe von "hinderlichen" Eigenschaften, wie geringeren Ertrag oder geringere Belastbarkeit bei Trockenheit in sich. Es müssen nun die Pflanzen identifiziert werden, welche, außer an den für die Resistenz verantwortlichen Stellen im Genom, den geringsten Anteil an Genom der Wildpflanze enthalten. Nur diese werden dann in der nachfolgenden Generation weiter verwendet.

Die Vorrichtung würde dieses Problem zum Beispiel auf die folgende Weise lösen.

Die Vorrichtung wird so ausgelegt, daß mit jeder Probenentnahme eine kleine (<1 cm Durchmesser) flexible Plastikmarke mit einer durchlaufenden Nummer durch das Blatt gestanzt wird, aus welchem die Probe entnommen wird. Die Plastikmarke kann auch statt dessen beim Entnahmeprozeß (von der Menge abhängig) von Hand aufgeklebt werden. Die Entnahmevorrichtung zählt bei jeder Entnahme mit, hat also jederzeit Kenntnis von den durchlaufenden Nummern und kann diese in einem Display anzeigen. Bei nicht-automatischem Befestigen der Markierung kann so der Bediener jederzeit kontrollieren, ob die Nummer, welche befestigt wird (oder wurde) auch der entspricht, welche die Entnahmevorrichtung dem jeweiligen Probenbehälter zuordnet. Da der Probenentnehmer ein Teil der Probenkapsel ist, erfolgt jede Entnahme so, daß kein Material von anderen Pflanzen mitgeschleppt wird und dadurch die nachfolgenden hochempfindlichen Analysen verunreinigt. Probenbehälter und -entnehmer werden bei dieser Variante zum Beispiel als lange Ketten von Einzeleinheiten oder in Magazinen geliefert und vor Beginn der Arbeiten in die Vorrichtung geladen. Dadurch können große Mengen von Proben hintereinander ohne neues Laden der Vorrichtung auf freiem Feld genommen werden. Die eigentliche Probenentnahme wird nun derart ausgeführt, daß der Bediener ein Blatt in den dafür vorgesehenen Zwischenraum der Vorrichtung einführt (dabei nur die Vorrichtung bewegend, also mit einer Hand ausgeführt) und mit Auslösen der Vorrichtung den Probendeckel durch das Blatt in den Probenbehälter stanzt. Der Deckel reißt eine definierte Menge des Gewebes des Blattes mit, welches direkt im durch den Vorgang verschlossenen Probenbehäter zu liegen kommt. Die gefüllten Probenkapsein können direkt, zum Beispiel in festzulegenden Einheitsmengen, aus der Vorrichtung entnommen werden und in einer Tragetasche gesammelt werden. Es ist auch möglich diese direkt im Magazin (welches ein Trommelmagazin sein kann) zu lagern. An einer Stelle der angelieferten Probenbehältereinheit ist eine Kodierung befestigt. Das heißt, daß wenn Probenbehülter in 24er Einheiten (z.B. Streifen die 24. fest aneinander gekettete Gefäße lang sind) angeliefert werden, eine Seriennummer, deren Position am Streifen auch die Richtung vorgibt, mit der dieser Streifen in die Vorrichtung eingeführt werden muß, die 24 nachfolgenden Gefäße eindeutig kennzeichnet. Die Seriennummer ist so geartet, daß mit an Sicherheit grenzender Wahrscheinlichkeit kein Anwender in einer Anwendung zweimal auf dieselbe Nummer stößt.

Bei der beispielhaften Anwendung bedeutet dies, daß nach der konkurrenzlos schnellen Entnahme und Markierung der Proben eine riesige Menge von Probebehältern zentral gesammelt werden. Diese liegen nun in 8er, 16er oder 24er Streifen vor, welche, an Hand der Seriennummer ausgerichtet, auf 96er - oder 384er Mikrotiterplattenformat verteilt werden. Dieses Format ist in der Molekularbiologie standardisiert. Die Seriennummern der Streifen können automatisch gelesen werden. Damit "weiß" eine Analysevorrichtung nun genau wo sich eine bestimmte Probe in der Mikrotiterplatte befindet.

Die Daten über die Pflanze (in diesem einfachen Fall die Nummer, mit welcher die Pflanze markiert wurde, aber auch jede beliebige andere Information wie zum Beispiel der Standort der Pflanze) befinden sich zunächst noch in oder bei der Vorrichtung. Eine Variante ist es nun, daß die Vorrichtung über ein Kabel mit einem Computer verbunden wird, welcher die Analysevorrichtung steuert oder nach der Analyse die Daten auswertet. Die Daten werden über ein in der Vorrichtung vorhandenes Interface auf einen Computer überspielt. Dieser kann nun jeder im 96er oder 384er Format gelagerten Probe genau die ihr entsprechenden Informationen zuordnen.

Nach der Analyse. weiche zentral durchgeführt wird, ist das Ziel genau die Nummer (und damit den Standort) der Pflanzen, welche dem gewünschten Genotyp am meisten entsprechen, festzustellen. In dem Fall, daß das Anbringen einer Nummer von der Vorrichtung erledigt wird hat während des gesamten Prozesses kein manueller Eingriff in den Datentransfer stattgefunden.

Die Fehleranfälligkeit und der Arbeitsaufwand bei einem solchen Verfahren werden dadurch minimal. Die beschriebene Lösung durch die vorgeschlagene Vorrichtung bedeutet also eine Kostensenkung.

### Beispiel zur Seuchenkontrolle bei Nutztieren

Es ist davon auszugehen, daß sich auch in Zukunft trotz aller Kontrollen infektiöse Krankheiten in Nutztierbeständen ausbreiten werden. Gerade im Falle einer Krankheit wie BSE, bei welcher verhindert werden muß, daß Fleisch infizierter Tiere gegessen wird, ist ein schnelles Testen von riesigen Zahlen von einzelnen Tieren notwendig. Außerdem sollten in Seuchenzeiten Importe und Exporte von Nutztieren überprüft werden. Dies bedeutet, daß Zoll und Grenzschutz in der Lage sein muß, Proben von großen Zahlen von Tieren zu nehmen und ohne größeren Verwaltungsaufwand zu registrieren und zur Analyse zu senden. Eine genaue Kontrolle darf aber nicht langwierig sein, da dies bei der verderblichen Ware zu wirtschaftlichen Verlusten der Tierzüchter führen würde

Eine Situation, in der die Vorrichtung zur Anwendung käme, wäre die, welche sich Ende der 80er Jahre in Großbritannien als reale Bedrohung erwiesen hat. Der gesamte Viehbestand eines großen Landes (auf den europäischen oder amerikanischen Kontinent bezogen wären noch viel größere Zahlen im Spiel) muß innerhalb kürzester Zeit auf eine Erkrankung getestet werden. Jedes kranke Tier könnte viele Todesopfer zur Folge haben, andererseits kann weder die Verwertung von Nutztieren gestoppt, noch alle Tiere notgeschlachtet werden. Im Falle der Bundesrepublik Deutschland würde dies das Testen großer Teile des Bestandes von 16 Millionen Rindern bedeuten. Für Schweine oder Geflügel wären diese Zahlen noch weit höher. Zur Zeit werden mit vielversprechenden Resultaten schnelle Tests für BSE entwickelt, für andere gefährliche Krankheiten gibt es schon solche Nachweise. In dem angedeuteten Fall würde ein großflächiges Testen des Tierbestandes hauptsächlich an der Logistik der Probenentnahme und deren Verwaltung scheitern. Tests sind nülzlich, wenn sie genau die infizierten Tiere identifizieren. Das vorgestellte Verfahren ist in der Lage dieses Problem zu lösen. Stattet man zum Beispiel die zahllosen Veterinäre mit je einer Vorrichtung und für solche Fälle vorrätigen Lieferungen an Probenbehältern aus, so kann praktisch ohne zusätzliche logistische oder Verwaltungsarbeit ein solches komplettes "screening" durchgeführt werden. Es muß lediglich sichergestellt werden, daß jeder beauftragte Veterinär einen bestimmten Zuständigkeitsbereich hat und daß jedes Landgut mit Viehbestand in den Zuständigkeitsbereich eines solchen Veterinärs fällt. Solche Zuständigkeiten sind für die allermeisten Herden längst durch bestehende Vorschriften geregelt. Im Falle einer Epidemie müßten die Veterinäre angewiesen werden in möglichst kurzer Zeit von allen Tiere im Zuständigkeitsbereich eine Probe zu nehmen. Die Variante der Vorrichtung würde dabei in etwa der entsprechen, welche in Figur 1 gezeigt wird. Eine Gewebeprobe enthält auch genug Blut um in praktisch allen Fällen die eigentliche, wesentlich aufwendigere Blutentnahme zu ersetzen. Der Veterinär muß vor dem Entnehmen einer Probe den Code des jeweiligen Tieres eingeben. Im Falle der fortschrittlichsten, bevorzugten Vorrichtungen würde dieser Code sogar an Hand der bei der ursprünglichen Probenentnahme (zur Erstellung eines genetischen Fingerabdrucks) hinterlassenen Ohrmarke (welche einen Transponder enthält) automatisch eingelesen. Ein besonderer Vorzug dieser Variante ist, daß eine Probe nur entnommen werden kann, wenn gleichzeitig mit der Probenentnahme ein Signal eines Transponder empfangen wird. Dieses Signal identifiziert ein Tier eindeutig und wird von der Vorrichtung zusammen mit allen anderen relevanten Daten der Probenkapsel zugeordnet, ohne daß dies vom Benutzer beeinflußt oder vermieden werden kann. Damit kann ausgeschlossen werden, daß die Herdbuchnummer eines kranken Tieres einer Herde eingelesen wird, dann aber die Probe von einem anderen, in diesem Fall gesunden Tier entnommen wird. Ein wesentlicher Vorteil der Vorrichtung ist die Kombination mit den erfindungsgemäßen Probenkapseln. Durch Verwendung von nur einmal verwendeten Probenentnehmern wird das Risiko einer Übertragung von Krankheiten minimiert. Die Sterilisierung einer Vorrichtung ist ein erheblicher Aufwand und müßte sehr gewissenhaft ausgeführt werden. Die erfindungsgemäße Vorrichtung löst diese Probleme auf einfachste Art und Weise.

In einer Krisensituation können von jedem Veterinär täglich hunderte von Proben entnommen werden, ohne daß dieser irgendwelche Proben beschriften oder sortieren müßte. Am Ende eines Tages können die Proben an eine zentrale Analysestation gesandt werden. Die Daten über die Proben werden entweder auf einem von der Vorrichtung separierbaren Speichermedium zusammen mit den Probenkapseln versandt oder über Telefon oder Internet getrennt übermittelt. In jedem Fall können die Proben im zentralen Labor direkt und vollautomatisch in einen Analyseprozeß integriert werden. Auch die Assoziation der Ergebnisse mit den Proben und damit den infizierten Beständen kann vollautomatisch erfolgen. Abgesehen von der sicheren Entnahme und Analyse bietet die Vorrichtung auch noch ein effizientes Werkzeug zur Seuchenkontralle. Durch den extrem schnellen Fluß von Daten in elektronischer Form können Seuchenherde und Verbreitungswege identifiziert werden, eine Voraussetzung für die Seuchenbekämpfung und damit Schadensminderung.

### Kurzbeschreibung der Figuren

Fig. 1 Vollautomatisch arbeitende Vorrichtung zur biologischen Probenentnahme. Die Vorrichtung führt nach einer Dateneingabe zwei Teile einer Probenkapsel unter Entnahme einer Gewebeprobe zusammen. Probenkapseln sind gegurtet. Jeder Probenkapselgurt hat ein Fahne mit einem einmaligen Code. Dieser Code wird von der Vorrichtung zur Registrierung gelesen und den Daten zugeführt.
Fig. 2 Einfache mechanisch Ausführung einer Vorrichtung zur biologischen Probenentnahme.
Fig. 3 Probenkapsel, die durch Stanzen eine Gewebeprobe entnehmen kann.
Fig. 4 Probenkapsel, die durch Schießen eine Gewebeprobe entnehmen kann.
Fig. 5 Probenkapsel, die durch Stoßen eine Gewebeprobe entnehmen kann.
Fig. 6 Probenkapsel, die durch Stechen eine Gewebeprobe entnehmen kann.
Fig. 7 Probenkapsel, die durch Kneifen eine Gewebeprobe entnehmen kann.
Fig. 8 Probenkapsel, die durch Kneifen eine Gewebeprobe entnehmen kann.
Fig. 9 Probenkapsel, die durch eine Biopsie eine Gewebeprobe entnehmen kann.
Fig. 10 Probenkapsel, die durch Kratzen eine Gewebeprobe entnehmen kann.
Fig. 11 Probenkapsel, die durch Kratzen eine Gewebeprobe entnehmen kann.
Fig. 12 Gegurteter Probenbehälterstreifen.
Fig. 13 Probendeckel in rotationssymmetrischer Form.
Fig. 14 Gestaitung von Probenbehälter und Probendeckel, mit welchen gleichzeitig eine Gewebeprobe entnommen und eine Ohrmarke angebracht werden können.
Fig. 15 Zugehöriges Gegenstück zu Fig. 14.
Fig. 16 Funktionsprinzip der Entnahme einer Gewebeprobe aus einem Rinderohr unter gleichzeitigem Anbringen einer Ohrmarke.
Fig. 17 Probenkapseldeckel, der während der Bewegung durch das Gewebe zuerst eine Probe entnimmt, dann eine scharfe Spitze bildet und das weiterdurchdrungene Gewebe möglichst wenig beschädigt.
Fig. 18 Variante von Fig. 17.
Fig. 19 Variante von Fig. 17.
Fig. 20 Probenkapseldeckel von Fig. 17 nach einer Gewebeprobeentnahme in der zugehörigen Probenkapsel.
Fig. 21 Funktionsweise des Probenkapseldeckels von Fig. 17.
Fig. 22 Tragbares Dateneingabegerät als Zusatzmodul zu einer Probenentnahmevorrichtung wie in Fig.2 gezeigt.
Fig. 23 Basisstation für des Dateneingabegerät von Fig. 22 mit welchem Daten übertragen werden können.
Fig. 24 Schema zum Verfahren des Identitätsnachweises einer Fleischprobe.
Fig. 25 Probenbchälter und Probendeckel in Kombination mit einer bei der Probenentnahme automatisch angebrachten Kennzeichnungsmarke wie sie zur Zeit bei Rindern benutzt werden. In der Marke integriert ist ein codierten Transpondersender.
Fig. 26 Zugehöriges Gegenstück zu Fig. 25 mit Transpondersender.

### Bezugszeichenliste

- 1: LCD-Display
- 2: numerische oder alphanumerische Tastatur
- 3: Probenbehältermagazin
- 4: Barcodelesestift
- 5: Chipkarte
- 6: Rändelmutter
- 7: Schußbolzen
- 8: Zwischenraum
- 9: Gegenhalter
- 10: Deckelmagazin
- 11: Griff
- 12: Auslöser
- 13: Gelenk
- 14: Zangenbacke mit Dorn
- 15: Probendeckel
- 16: Zangenbacke mit Bohrung
- 17: Probenbehälter
- 18: Drchpunkt
- 19: Feder
- 20: linker Zangengriff
- 21: rechter Zangengriff
- 22: Probenbehälter
- 23: Probenkapselverschluß
- 24: Aussparung
- 25: scharfe Außenkante
- 26: Vertiefung
- 27: Nut
- 28: Probenkapseldeckel
- 29: Probenbehälter
- 30: Vertiefung
- 31: scharlkantige Nut
- 32: Probenkapseldeckel
- 33: Ansatz
- 34: Vertiefung
- 35: Probenbehälter
- 36: Septum
- 37: Probenkapscldeckel
- 38: Spitze
- 39: Widerhaken
- 40: Probenbehälter
- 41: Probenkapseldeckel
- 42: Ansatz.
- 43: spitz zulaufende Nase
- 44: Probenbehälter
- 45: Probenbehälter
- 46: Deckel
- 47: Gelenk
- 48: probenehmender Teil
- 49: schneidende Kante
- 50: Probenbehälter
- 51: Deckel
- 52: Probenkapscldeckel
- 53: Probenbehälter
- 54: Lamellen oder Zähne
- 55: Anschlag
- 56: Probenkapscldeckel
- 57: Probenbehälter
- 58: Kratzer
- 59: Scheibe mit Innenverzahnung
- 60: Probenbchälter
- 61: Steg
- 62: Verzahnung
- 63: Scheibe mit zylindrischem Ansatz
- 64: Probendeckel
- 65: Nut
- 66: Transponder
- 67: Probengewebe (Ohr)
- 68: ein Schlitz
- 69: Halbrund
- 70: Bohrung
- 71: Nut
- 72: zwei Schlitze
- 73: drei Schlitze
- 74: trapezförmig
- 75: Probennchmer
- 76: Probenbehälter
- 77: entnommenes Gewebe
- 78: LCD-Display
- 79: Tastatur
- 80: Schnittstelle
- 81: Battericeinheit
- 82: Basisstation
- 83: Netzkabel
- 84: Telefonkabel
- 85: Scheibe bzw. Marke mit Innenverzahnung
- 86: Probenbehälter
- 87: Steg
- 88: Verzahnung
- 89: Scheibe bzw. Marke mit zylindrischem Ansatz
- 90: Probendeckel
- 91: Nut
- 92: Transponder

## Patentansprüche

1. Vorrichtung mit mindestes einem Proben Kapseldeckel (64,90) und mindesten einem Probenbehälter (60, 86) zur Entnahme von biologischen Proben, wobei
- die Vorrichtung über eine Aufnahme (10,14) verfügt, welche in der Lage ist einen oder mehrere Probenkapseldeckel aufzunehmen,
- die Vorrichtung über eine weitere Aufnahme (3,16) verfügt, welche in der Lage ist einen oder mehrere Probenbehälter aufzunehmen,
- die Vorrichtung über eine Mechanik (7,9,12,18-21) verfügt, welche den Probenkapseldeckel und den Probenbehälter in einem Arbeitsgang mit der Entnahme einer biologischen Probe entweder durch den Probenkapseldeckel oder den Probenbehälter zur Probenkapsel zusammenfügt, wobei entweder die Probenkapsel ein oder mehrere Teile (59,63) umfaßt, die beim Eintnehmender Probe abgetrennt werden konnen im als Markierung im Gewebe des Lebewesens zu verbleiben, oder ein oder mehrere Bestandteile (85,89) der Probenkapsel nach dem Zusammenfügen der Probenkapsel eine Ohrmarke ergeben die von der Probenkapsel abgetrennt werden kann um als Markierung im Gewebe des Lebewesens zu verbleiben.

2. Vorrichtung nach Anspruch 1), **dadurch gekennzeichnet, daß** diese über eine Mechanik verfügt, welche nach dem Ansetzen der Vorrichtung an ein Gewebe und Betätigung eines Auslösemechanismus einen Bestandteil der Probenkapsel mittels einer Art Schlagbolzen derart durch das Gewebe stanzt oder schießt, daß auf dem Wege durch das Gewebe eine Gewebeprobe entnommen wird und der Bestandteil im selben Bewegungsablauf mit einem weiteren Teil der Probenkapsel zur geschlossenen Probenkapsel zusammengeführt wird.

3. Vorrichtung nach Anspruch 1), **dadurch gekennzeichnet, daß**
- diese ein oder mehr Magazine enthält, in welche jeweils einzelne oder zu Streifen verkettete Probenkapseldeckel, Probenbehälter und Teile von Markierungen geladen werden können und
- über eine Mechanik verfügt, welche diese Bestandteile einer Probenkapsel durch Betätigung eines Auslösemechanismus gemeinsam innerhalb der Magazine um eine Position in Richtung eines Probenentnahmemechanismus der Vorrichtung verschiebt.

4. Vorrichtung nach Anspruch 1), **dadurch gekennzeichnet, daß** diese über einen Mechanismus verfügt, welcher
- in einem ersten Schritt den probenentnehmenden Teil der Probenkapsel beim Ansetzen der Vorrichtung an das Gewebe und nach Betätigung eines Auslösemechanismus derart zusammenpreßt, daß durch eine kneifende Bewegung eine Gewebeprobe entnommen wird,
- oder in einem ersten Schritt den probenentnehmenden Teil der Probenkapsel beim Ansetzen der Vorrichtung an das Gewebe und nach Betätigung des Auslösemechanismus derart am Gewebe vorbeiführt, daß durch eine kratzende Bewegung eine Gewebeprobe entnommen wird.
- oder in einem ersten Schritt den probenentnehmenden Teil der Probenkapsel beim Ansetzen der Vorrichtung an das Gewebe und nach Betätigung des Auslösemechanismus derart unter Zusammenquetschen des Probenentnehmers am Gewebe vorbeiführt, daß Haare ausgerissen werden,
- und in einem weiteren Schritt des selben Bewegungsablaufs oder ein nochmaliges Betätigen des Auslösemechanismus den Probenbehälter mit Probenkapseldeckel zur Probenkapsel zusammenfügt.

5. Vorrichtung nach Anspruch 1), **dadurch gekennzeichnet, daß**
- ein den Probenbehälter führender Teil der Vorrichtung und ein den Probenkapseldeckel führender Teil der Vorrichtung derart von entgegengeseizten Seiten fest an ein Gewebe gedrückt werden können, daß ein sich auf einer der beiden Seiten befindlicher Mechanismus einen Teil der Probenkapsel zu einer Probenentnahme verwenden kann und
- der so ausgeübte Druck und der Abstand zwischen dem den Probenbehälter führenden Teil der Vorrichtung und der den Probenkapseldeckel führenden Teil der Vorrichtung durch eine Verstellschraube oder analoge Vorrichtung reguliert werden kann.

6. Vorrichtung nach Anspruch 1), **dadurch gekennzeichnet, daß** sie über eine Mechanik verfügt, welche die Probenkapseldeckel von einem Magazin aus auf die Achse eines Schußbolzens und die Probenbehälter von einem Magazin aus auf die Zielseite der Vorrichtung vorlegt.

7. Vorrichtung nach Anspruch I), **dadurch gekennzeichnet, daß** die Vorrichtung über eine Möglichkeit zur Dateneingabe und/oder zur Datenausgabe verfügt oder an eine solche gekoppelt ist.

8. Vorrichtung nach Anspruch 1) **dadurch gekennzeichnet, daß** die Vorrichtung über eine Mechanik und/oder Elektronik, verfügt mittels derer automatisch Daten wie
- die Seriennummer der Probenbehälter oder mehrerer verketteter Probenbehälter und/oder
- Informationen über das Lebewesen, dessen Probe entnommen wird und/oder
- andere Informationen, welche die Probenentnahme betreffen eingelesen werden.

9. Vorrichtung nach Anspruch 1), **dadurch gekennzeichnet, daß** sie über eine numerische oder alphanumerische Tastatur und/oder eine Einrichtung zum Empfang oder zum Lesen von Daten verfügt.

10. Vorrichtung nach Anspruch 1) **dadurch gekennzeichnet, daß** diese über ein Display verfügt, in welchem eingelesene und von der Vorrichtung bereitgestellte Daten angezeigt werden.

11. Vorrichtung nach Anspruch 1), **dadurch gekennzeichnet, daß** eine Mechanik zur Auslösung durch eine weitere Mechanik blockiert ist, welche nur durch vorherige Eingabe oder Bestätigung von Daten freigegeben wird.

12. Vorrichtung nach Anspruch 1) **dadurch gekennzeichnet, daß**
- diese über eine Elektronk verfügt, die eingegebene oder automatisch eingelesene Seriennummern der Probenkapseln und eingegebene oder automatisch eingelesene Informationen über das Lebewesen, dessen Probe entnommen wurde und andere, die jeweilige Probenentnahme betreffende Informationen assoziierent und
- diese Daten samt der Information über die Assoziation dieser Daten auf einem Speichermedium ablegt.

13. Vorrichtung nach Anspruch 12) **dadurch gekennzeichnet, daß** das Speichermedium a) fest in der Vorrichtung installiert ist oder b) von dieser abtrennbar ist.

14. Vorrichtung nach Anspruch 12) **dadurch gekennzeichnet, daß** diese eine Elektronik umfaßt mittels derer alle gespeicherten Daten und Informationen über die Assoziation dieser Daten drahtlos ühermittelt werden und gegebenenfalls auch Daten drahtlos empfangen werden.

15. Vorrichtung nach Anspruch 12) **dadurch gekennzeichnet, daß** diese über eine Schnittstelle verfügt, über welche eine Kabelverbindung zwecks Datenübertragung von oder zur Vorrichtung hergestellt werden kann.

16. Vorrichtung nach Anspruch 1), **dadurch gekennzeichnet, daß** diese über einen Mechanismus verfügt, welcher gekoppelt an die Probenentnahme eine Markierung am Lebewesen anbringt, die entweder aus a) einer einfachen farblichen Markierung b) einer selbstklebenden Folie c) einer haftenden Plakette oder d) einer selbsthaftenden oder fest im Gewebe verankerten Vorrichtung besteht, welche als Träger entweder
- eines Barkodes,
- eines lesbaren und/oder beschreibbaren integrierten Schaltkreises,
- eines Magnetstreifens,
- eines Transponders,
- eines Senders,
- eines Nummernkodes,
- eines Buchstabenkodes oder
- eines vergleichbaren Kodierungs oder Informationsträgersystems
- oder einer einfachen farblichen Markierung dient.

17. Vorrichtung nach Anspruch 1), **dadurch gekennzeichnet, daß** diese über eine Mechanik verfügt, welche die Vorrichtung nur bei gleichzeitigem Empfang des Signals eines Transponders freigibt.

18. Probenkapsel mit einem Probenbehälter (60) und einem Proben Kapseldeckel wobei der Probenbehälter und der Probenkapseldeckel in einem Arbeitsgang mit der Entnahme der Probe entweder durch den Probenkapseldeckel oder den Probenbehälter zusammenfügbar sind, und wobei sie einen oder mehrere Teile (59, 53) umfaßt, die beim Entnahmen der Probe abgetrennt werden können um als Markierung im Gewebe des Lebewesens zu verbleiben.

19. Probenkapsel mit einem Probenbehälter (86) und einem Probenkapseldeckel (90), wobei der Probenbehälter und der Probenkapseldeckel in einem Arbeitsgang mit der Entnahme der Probe entweder durch den Probenkapseldeckel oder den Probenbehälter zusammenfügbar sind, und wobei ein oder mehrere Bestandteile (85,89) der Probenkapsel nach dem Zusammenfügen der Probenkapsel eine Ohrmarke ergeben die von der Probenkapsel abgetrennt werden kann um als Markierung im Gewebe des Lebewesens zu verbleiben.

20. Probenkapsel nach Anspruch 18 oder 19, **dadurch gekennzeichnet, daß** ein Bestandteil der Probenkapsel zur Entnahme einer Gewebeprobe durch Stanzen, Schießen, Kratzen, Kneifen, Stoßen, durch Ausreißen von Haaren oder Ausführen der Bewegung einer Biopsienadel verwendet wird.

21. Probenkapsel nach Anspruch 18 oder 19), **dadurch gekennzeichnet, daß**
- einer oder mehrere identische Bestandteile solcher Probenkapseln miteinander verkettet sind,
- die entstehenden Streifen oder Ringe von Teilen von Probenkapseln als Einheiten von mehr als nur einem solchen Teil einer Probenkapsel in das Magazin oder einen analogen Teil einer Vorrichtung geladen werden können
- die Richtung, in der diese Streifen oder Ringe mehrerer solcher Einheiten, in die Vorrichtung geladen werden, durch eine an nur einer Stelle eines solchen Streifens oder Ringes angebrachten Markierung festgelegt sind und
- Seriennummern aller so verketteten Teile von Probenkapseln durch nur eine Scriennummer in eindeutiger Art und Weise definiert werden.

22. Probenkapsel nach Anspruch 21), **dadurch gekennzeichnet, daß** die Seriennummer, welche auf diesen Streifen oder Ringen angebracht ist, eine automatisch von einer dafür vorgesehenen Vorrichtung lesbare Form hat oder vom Benutzer selber gelesen und manuell in eine dafür vorgesehene Vorrichtung eingehen werden muß.

23. Prohenkapsel nach Anspruch 18 oder 19) **dadurch gekennzeichnet, daß** sie eine Stelle aufweist, welche das Durchstoßen mit Nadeln, Bolzen, Kanülen oder vergleichbaren Vorrichtungen z.B. durch ein Septum hindurch erlaubt.

24. Probenkapsel nach Anspruch 18 oder 19), **dadurch gekennzeichnet, daß** einer der Teile der Probenkapsel, welcher im Gewebe des Lebewesens, dessen Probe entnommen wurde verbleibt
- einen Barkode,
- einen lesbaren und/oder beschreibbaren integrierten Schaltkreise,
- einen Magnetstreifen,
- einen Transponder,
- einen Sender,
- einen Nummernkode,
- einen Buchstabenkode oder
- eine vergleichbare Kodierungs oder Informationsträgersystem
- oder eine einfache farbliche oder analoge Markierung umfaßt.

25. Probenkapsel nach Anspruch 19), **dadurch gekennzeichnet, daß** die im Gewebe verbleibende Ohrmarke nur unter Hinterlassung von sichtbaren Beschädigungen an entweder Ohrmarke oder Lebewesen entfernt werden kann.

26. Probenkapsel nach Anspruch 18 oder 19), **dadurch gekennzeichnet, daß** der Probenkapseldeckel als Probennehmer auf der probenabgewandten Seite eine Bohrung, einen Schlitz oder ähnliches zur Fixierung einer Führung besitzt.

27. Probenkapsel nach Anspruch 18 oder 19), **dadurch gekennzeichnet, daß** der probenentnehmende Teil der Probenkapsel auf der der Probe abgewandten Seite Vertiefungen aufweist, welche dazu dienen den Probenentnehmer a) derart an das Gewebe zu pressen und zu quetschen, daß dadurch kueifend eine Gewebeprobe entnommen wird oder b) derart schräg und unter Druck am Gewebe vorbeizuführen, daß kratzend eine Gewebeprobe entnommen wird.

28. Probenkapsel nach Anspruch 18 oder 19), **dadurch gekennzeichnet, daß** der Probenkapseldeckel auf der probenabgewandten Seite einen Fortsatz zu dessen leichter Entfernung aus dem Probenbehälter aufweist, allerdings nicht ohne Spuren an der Kapsel zu hinterlassen.

29. Probenkapsel nach Anspruch 18 oder 19) **dadurch gekennzeichnet, daß** der Probenkapseldeckel als Probennehmer auf der probenzugewandten Seite einen oder mehrere Schlitze, Bohrungen oder Vertiefungen zur Aufnahme von Gewebe aufweist.

30. Probenkapsel nach Auspruch 18 oder 19) **dadurch gekennzeichnet, daß** der Probenkapseldeckel geschlitzt und zum Teil konisch ist, damit zwei oder mehrere Spitzen bei Berührung des Probanden leicht in ihn hineingestochen werden und dann durch die konische Form zusammengedrückt werden, so daß eine geringe Probenmenge abgeschert bzw. geschnitten wird und kein weiteres Material mehr entnommen wird.

31. Vorrichtung bzw. Probenkapsel nach Anspruch 1, 18 oder 19 **dadurch gekennzeichnet, daß** der Probenbehälter nach dem Verschließen durch den Probenkapseldeckel nur unter Hinterlassen feststellbarer Veränderungen wieder geöffnet oder deren Inhalt erreicht werden kann.

32. Vorrichtung Anspruch 1) **dadurch gekennzeichnet, daß** einzelne oder mehrere in einer Kette zusammengefaßte Probenbehälter über eine Seriennummer verfügen.

33. Vorrichtung bzw. Probenkapsel nach Anspruch 1, 18 oder 19) **dadurch gekennzeichnet, daß** Teile der Probenkapsel mit zur weiteren Verarbeitung der Probe benötigten Reagenzien gefüllt sind.

## Claims

1. Device with at least one sample capsule cover (64, 90) and at least one sample container (60, 86) for withdrawing biological samples, wherein
- the device is provided with a receptacle (10,14), which can receive one or more sample capsule covers,
- the device is provided with another receptacle (3, 16), which can receive one or more sample containers;
- the device is provided with a mechanism (7, 9,12, 18-21), which joins the sample capsule cover and the sample container in one working cycle with the removal of a biological sample either by the sample capsule cover or by the sample container to form the sample capsule, wherein either the sample capsule comprises one or more parts (59, 63), which can be separated upon withdrawing the sample, in order to remain as a labelling in the tissue of the animal, or one or more components (85, 89) of the sample capsule produce an ear mark after the sample capsule has been joined, which can be separated from the sample capsule in order to remain as labelling in the tissue of the living organism.

2. Device according to claim 1, **characterized in that** the device is provided with a mechanism, which punches or shoots a component of the sample capsule by means of a type of firing pin through the tissue, after placement of the device on a tissue and actuating a trigger mechanism, in such a way that a tissue sample is removed on the path through the tissue and the component is brought together with another part of the sample capsule in the same motion cycle to produce a closed sample capsule.

3. Device according to claim 1, **characterized in that**
- the latter contains one or more magazines, in which sample capsule covers, sample containers and parts of labellings can be loaded either individually or interconnected to strips, and
- is provided with a mechanism, which moves these components of a sample capsule, by actuating a trigger mechanism jointly within the magazine, by one position in the direction of a sample-removing mechanism of the device.

4. Device according to claim 1, **characterized in that** the latter is provided with a mechanism, which
- in a first step, presses together the sample-removing part of the sample capsule upon placing the device against the tissue and after actuating a trigger mechanism, in such a way that a tissue sample is removed by a pinching motion;
- or, in a first step, upon applying the device on the tissue and after actuating the trigger mechanism, advances the sample-removing part of the sample capsule on the tissue such that
a tissue sample is removed by a scraping motion;
- or, in a first step, upon applying the device on the tissue and after actuating the trigger mechanism, advances the sample-removing part of the sample capsule while squeezing together the sample remover past the tissue in such a way that hairs are torn out;
- and in a further step of the same motion cycle or an additional actuating of the trigger mechanism joins the sample container with the sample capsule cover to form the sample capsule.

5. Device according to claim 1, **characterized in that**
- a part of the device bearing the sample container and a part of the device bearing the sample capsule cover can be pressed from opposite sides solidly onto the tissue, in such a way that a mechanism present on one of the two sides can use a part of the sample capsule for a sample removal, and
- the pressure thus exercised and the distance between the part of the device bearing the sample container and the part of the device bearing the sample capsule cover can be regulated by an adjusting screw or analogous device.

6. Device according to claim 1, **characterized in that** it is provided with a mechanism, which takes the sample capsule cover from a magazine and places it onto the axis of a firing pin and places the sample container of a magazine on the target side of the device.

7. Device according to claim 1, **characterized in that** the device provides a possibility for data entry and/or for data output or is coupled to such a device.

8. Device according to claim 1, **characterized in that** the device is provided with a mechanism and/or electronics, by means of which, data such as
- the serial number of the sample container or several sample containers linked together and/ or
- information on the organism, whose sample is removed, and/or
- other information, which concerns the sample removal, are read in automatically.

9. Device according to claim 1, **characterized in that** it is provided with a numerical or alphanumerical keyboard and/or a device for receiving or for reading of data.

10. Device according to claim 1, **characterized in that** the latter is provided with a display, in which data that are read in and processed by the device are displayed.

11. Device according to claim 1, **characterized in that** a trigger mechanism is blocked by another mechanism, which is released only by the prior input or confirmation of data.

12. Device according to claim 1, **characterized in that**
- the device is provided with electronics that associates the entered or automatically read-in serial numbers of the sample capsules and the entered or automatically read-in information on
the living organism, whose sample has been taken, and other information concerning the respective sample removal; and
- storing these data on a storage medium along with the information on the association of these
data.

13. Device according to claim 12, **characterized in that** the storage medium is a) installed rigidly in the device or b) can be separated from it.

14. Device according to claim 12, **characterized in that** it includes electronics, by means of which all stored data and information on the association of these data can be transmitted in a wireless manner and data can also be received, if need be, in a wireless manner.

15. Device according to claim 12, **characterized in that** the device is provided with an interface, by means of which a cable connection can be produced for purposes of data transmission from or to the device.

16. Device according to claim 1, **characterized in that** the device is provided with a mechanism, which affixes a labelling on the living organism, coupled with a removal of sample, which consists either of
a) a simple colour label, or
b) a self-adhering foil,
c) an adhering badge, or
d) a self-adhering device or one that is rigidly anchored in the tissue, which serves as a carrier either of
- a barcode,
- a readable and/or writable integrated circuit,
- a magnetic strip,
- a transponder,
- a transmitter,
- a numerical code,
- a letter code, or
- a comparable coding or information carrier system
- or a simple colour labelling.

17. Device according to claim 1, **characterized in that** the device is provided with a mechanism, which releases the device only with the simultaneous receiving of the signal of a transponder.

18. Sample capsule with a sample container (60) and a sample capsule cover (64), wherein the sample container and the sample capsule cover are joinable in a working cycle with the withdrawal of the sample either by the sample capsule cover or by the sample container, wherein the sample capsule comprises one or more parts (59, 63), which can be separated upon withdrawing the sample, in order to remain as a labelling in the tissue of the animal.

19. Sample capsule with a sample container (86) and a sample capsule cover (90), wherein the sample container and the sample capsule cover are joinable in a working cycle with the withdrawal of the sample either by the sample capsule cover or by the sample container, wherein one or more components (85, 89) of the sample capsule produce an ear mark after the sample capsule has been joined, which can be separated from the sample capsule in order to remain as a labelling in the tissue of the living organism.

20. Sample capsule according to claim 18 or 19, **characterized in that** a component of the sample capsule is used for removing a tissue sample by punching, shooting, scraping, pinching, guided shooting, by tearing out hairs, or conducting the movement of a biopsy needle.

21. Sample capsule according to claim 18 or 19, **characterized in that**
- one or more identical components of such sample capsules are linked with one another;
- the strips or rings that are formed of parts of sample capsules can be loaded into the magazine or into an analogous part of a device as units of more than only one such part of a sample capsule;
- the direction in which these strips or rings of several such units are loaded into the device is established by a labelling introduced on only one site of such a strip or ring;
- serial numbers of all such linked parts of sample capsules are defined by only this one serial
number in a clear manner.

22. Sample capsule according to claim 21, **characterized in that** the serial number, which is applied onto these strips or rings has a form that is automatically readable by a device provided for this purpose or must be read by the user himself and manually entered into a device provided for this purpose.

23. Sample capsule according to claim 18 or 19, **characterized in that** it has a site, which permits piercing with needles, pins, cannula or comparable devices, e.g., through a septum.

24. Sample capsule according to claim 18 or 19, **characterized in that** one of the parts of the sample capsule, which remains in the tissue of the living organism whose sample was removed, includes
- a barcode,
- the readable and/or writable integrated circuit,
- a magnetic strip,
- a transponder,
- a transmitter,
- a numerical code,
- a letter code, or
- a comparable coding or information carrier system
- or a simple colour or analogous labelling.

25. Sample capsule according to claim 19, **characterized in that** the ear mark remaining in the tissue can be removed only by leaving behind visible damage to either the ear mark or the living organism.

26. Sample capsule according to claim 18 or 19, **characterized in that** the sample capsule cover as sample taker has a bore, a slot, or the like on the side turned away from the sample for attaching of a guide.

27. Sample capsule according to claim 18 or 19, **characterized in that** the sample-removing part of the sample capsule on the side turned away from the sample has depressions, which serve for the purpose of pressing and squeezing the sample remover a) at the tissue in such a way that a tissue sample is removed by pinching or b) advancing it obliquely and under pressure at the tissue in such a way that a tissue sample is removed by scraping.

28. Sample capsule according to claim 18 or 19, **characterized in that** the sample capsule cover on the side turned away from the sample has an extension piece for its easy removal from the sample container, of course not without leaving behind traces on the capsule.

29. Sample capsule according to claim 18 or 19, **characterized in that** the sample capsule cover as a sample taker has one of more slots, bores or depressions for receiving tissue on the side turned toward the sample.

30. Sample capsule according to claim 18 or 19, **characterized in that** the sample capsule cover is slotted and partly conical, so that two or more tips will be easily punched into the sample subject upon contacting the sample subject, and then are pressed together due to the conical shape, so that a small quantity of sample is sheared off or cut and no additional material will be removed.

31. Device or sample capsule according to claim 1, 18 or 19, **characterized in that** the sample container, after it has been sealed by the sample capsule cover, can be opened again or its contents can be reached only by leaving behind changes that can be discerned.

32. Device according to claim 1, **characterized in that** individual sample containers or several sample containers joined together in a chain are provided with a serial number.

33. Device or sample capsule according to claim 1, 18 or 19, **characterized in that** parts of the sample capsule are filled with reagents necessary for the further processing of the sample.

## Revendications

1. Dispositif comprenant au moins un capuchon (64, 90) de capsule d'échantillon et au moins un tube d'échantillon (60, 86) pour le prélèvement d'échantillons biologiques, sachant que
- le dispositif comporte un logement (10, 14) qui est apte à recevoir un ou plusieurs capuchons de capsule d'échantillon,
- le dispositif comporte un logement (3, 16) supplémentaire qui est apte à recevoir un ou plusieurs tubes d'échantillon,
- le dispositif comporte un mécanisme (7, 9, 12, 18-21) qui, au cours d'un processus de travail incluant le prélèvement d'un échantillon biologique soit au moyen du capuchon de capsule d'échantillon, soit au moyen du tube d'échantillon, assemble le capuchon de capsule d'échantillon et le tube d'échantillon pour former une capsule d'échantillon, sachant que soit la capsule d'échantillon comporte une ou plusieurs parties (59, 63) qui, au prélèvement de l'échantillon, peuvent être séparées pour rester sous forme de marquage dans le tissu de l'être vivant, soit un ou plusieurs composants (85, 89) de la capsule d'échantillon donnent après l'assemblage de la capsule d'échantillon une marque d'oreille qui peut être séparée de la capsule d'échantillon pour rester sous forme de marquage dans le tissu de l'être vivant.

2. Dispositif selon la revendication 1, **caractérisé en ce que** ledit dispositif comporte un mécanisme qui, après la pose du dispositif sur un tissu et la manipulation d'un mécanisme de déclenchement, à la manière d'une tige de percussion, presse ou projette un composant de la capsule d'échantillon à travers le tissu de manière à prélever un échantillon de tissu sur la trajectoire à travers le tissu et le composant, au cours du même déplacement, est assemblé avec une autre partie de la capsule d'échantillon pour former une capsule d'échantillon fermée.

3. Dispositif selon la revendication 1, **caractérisé en ce que** ledit dispositif comporte
- un ou plusieurs magasins, dans lesquels peuvent être chargés, séparément ou assemblés en chaîne, des capuchons de capsule d'échantillon, des tubes d'échantillon et des parties de marquage, et
- un mécanisme qui, par la manipulation d'un mécanisme de déclenchement, déplace conjointement les composants d'une capsule d'échantillon à l'intérieur des magasins d'une position en direction d'un mécanisme de prélèvement d'échantillons du dispositif.

4. Dispositif selon la revendication 1, **caractérisé en ce que** ledit dispositif comporte un mécanisme, lequel
- au cours d'une première étape, au moment de la pose du dispositif contre le tissu et après la manipulation d'un mécanisme de déclenchement, comprime la partie de la capsule prélevant l'échantillon, de telle sorte qu'un échantillon de tissu est prélevé par un mouvement de pincement,
- ou, au cours d'une première étape, au moment de la pose du dispositif contre le tissu et après la manipulation d'un mécanisme de déclenchement, déplace le long du tissu la partie de la capsule prélevant l'échantillon, de telle sorte qu'un échantillon de tissu est prélevé par un mouvement de grattage,
- ou, au cours d'une première étape, au moment de la pose du dispositif contre le tissu et après la manipulation d'un mécanisme de déclenchement, déplace le long du tissu la partie de la capsule prélevant l'échantillon, en comprimant l'élément de prélèvement de manière à arracher des cheveux,
- et, au cours d'une étape suivante du même processus de déplacement ou après une nouvelle manipulation d'un mécanisme de déclenchement, assemble le tube d'échantillon avec le capuchon de capsule d'échantillon pour former une capsule d'échantillon.

5. Dispositif selon la revendication 1, **caractérisé en ce que**
- une partie du dispositif guidant le tube d'échantillon et une partie du dispositif guidant le capuchon de capsule d'échantillon peuvent être pressées à partir de deux côtés opposés fermement contre un tissu, de telle sorte qu'un mécanisme agencé sur l'un des deux côtés peut utiliser une partie de la capsule d'échantillon pour un prélèvement d'échantillon, et
- la pression ainsi exercée et la distance entre la partie du dispositif guidant le tube d'échantillon et la partie du dispositif guidant le capuchon de capsule d'échantillon peuvent être régulées par une vis de réglage ou un dispositif analogue.

6. Dispositif selon la revendication 1, **caractérisé en ce que** ledit dispositif comporte un mécanisme, qui pose le capuchon de capsule d'échantillon à partir d'un magasin sur l'axe d'une tige de percussion et pose les tubes d'échantillon à partir d'un magasin sur le côté cible du dispositif.

7. Dispositif selon la revendication 1, **caractérisé en ce que** ledit dispositif comporte une possibilité de saisie de données et/ou d'édition de données ou est raccordé à une telle unité.

8. Dispositif selon la revendication 1, **caractérisé en ce que** ledit dispositif comporte un mécanisme et/ou un dispositif électronique, qui permettent d'enregistrer automatiquement des données, telles que
- le numéro de série du tube d'échantillon ou de plusieurs tubes d'échantillons reliés en chaîne, et/ou
- des informations relatives à l'être vivant sur lequel on prélève des échantillons, et/ou
- d'autres informations qui concernent le prélèvement d'échantillons.

9. Dispositif selon la revendication 1, **caractérisé en ce que** ledit dispositif comporte un clavier numérique ou alphanumérique et/ou une unité pour recevoir ou pour lire des données.

10. Dispositif selon la revendication 1, **caractérisé en ce que** ledit dispositif comporte un écran, sur lequel sont affichées des données entrées ou préparées par le dispositif.

11. Dispositif selon la revendication 1, **caractérisé en ce qu'**un mécanisme de déclenchement est bloqué par un autre mécanisme, qui est activé uniquement par la saisie préalable de données ou la validation de celles-ci.

12. Dispositif selon la revendication 1, **caractérisé en ce que**
- ledit dispositif comporte une unité électronique, qui associe des numéros de série des capsules d'échantillon saisis ou chargés automatiquement, des informations saisies ou chargées automatiquement, relatives à l'être vivant sur lequel on prélève des échantillons et d'autres informations qui concernent le prélèvement d'échantillons correspondant, et
- stocke ces données, y compris l'information sur l'association de ces données dans un support de mémoire.

13. Dispositif selon la revendication 12, **caractérisé en ce que** le support de mémoire est a) installé de manière fixe dans le dispositif ou b) peut être séparé de celui-ci.

14. Dispositif selon la revendication 12, **caractérisé en ce que** ledit dispositif comporte une unité électronique, au moyen de laquelle toutes les données et informations sur l'association de ces données, stockées en mémoire, sont transmises sans fil et, le cas échéant, des données sont aussi reçues sans fil.

15. Dispositif selon la revendication 12, **caractérisé en ce que** ledit dispositif comporte une interface, au moyen de laquelle on peut établir une liaison par câble pour la transmission de données à partir du dispositif ou vers le dispositif.

16. Dispositif selon la revendication 1, **caractérisé en ce que** ledit dispositif comporte un mécanisme qui, couplé à l'unité de prélèvement d'échantillon, applique un marquage sur l'être vivant, qui est formé soit a) par un simple repère de couleur, soit b) par une feuille autocollante, soit c) par une plaquette adhésive, soit d) par un dispositif autocollant ou ancré fermement dans le tissu, lequel est utilisé comme support
- soit d'un code à barres,
- soit d'un circuit intégré, dans lequel on peut lire ou écrire,
- soit d'une bande magnétique,
- soit d'un transpondeur,
- soit d'un émetteur,
- soit d'un code à chiffres,
- soit d'un code à lettres,
- soit d'un codage similaire ou système de support d'informations,
- soit d'un simple repère de couleur.

17. Dispositif selon la revendication 1, **caractérisé en ce que** ledit dispositif comporte un mécanisme qui active le dispositif uniquement à la suite de la réception simultanée du signal d'un transpondeur.

18. Capsule d'échantillon comprenant un tube d'échantillon (60) et un capuchon de capsule d'échantillon, le tube d'échantillon et le capuchon de capsule d'échantillon pouvant être assemblés au cours d'un processus de travail incluant le prélèvement d'un échantillon soit au moyen du capuchon de capsule d'échantillon, soit au moyen du tube d'échantillon, et sachant que ladite capsule comprend une ou plusieurs parties (59, 63) qui, au prélèvement de l'échantillon, peuvent être séparées pour rester sous forme de marquage dans le tissu de l'être vivant.

19. Capsule d'échantillon comprenant un tube d'échantillon (86) et un capuchon de capsule d'échantillon (90), le tube d'échantillon et le capuchon de capsule d'échantillon pouvant être assemblés au cours d'un processus de travail incluant le prélèvement d'un échantillon soit au moyen du capuchon de capsule d'échantillon, soit au moyen du tube d'échantillon, et sachant qu'un ou plusieurs composants (85, 89) de la capsule d'échantillon donnent après l'assemblage de la capsule d'échantillon une marque d'oreille qui peut être séparée de la capsule d'échantillon pour rester sous forme de marquage dans le tissu de l'être vivant.

20. Capsule d'échantillon selon la revendication 18 ou 19, **caractérisée en ce qu'**un composant de la capsule d'échantillon est utilisé pour le prélèvement d'un échantillon de tissu par découpe, projection, grattage, pincement, ponction, par arrachage de cheveux ou exécution du mouvement d'une aiguille de biopsie.

21. Capsule d'échantillon selon la revendication 18 ou 19, **caractérisée en ce que**
- un ou plusieurs composants identiques de telles capsules d'échantillon sont reliés en chaîne les uns aux autres,
- les bandes ou anneaux obtenus avec les parties des capsules d'échantillon peuvent être chargées, sous forme d'unités de plus d'un tel élément d'une capsule d'échantillon, dans un magasin ou une partie analogue du dispositif,
- la direction dans laquelle ces bandes ou anneaux formés d'une pluralité de tels éléments sont chargés dans le dispositif, est définie par un repère appliqué à un seul emplacement d'une telle bande ou d'un tel anneau, et
- des numéros de série de tous les éléments des capsules d'échantillon ainsi reliés en chaîne sont définis de manière non équivoque par un seul numéro de série.

22. Capsule d'échantillon selon la revendication 21, **caractérisée en ce que** le numéro de série, qui est appliqué sur ces bandes ou anneaux, a une forme pouvant être lue automatiquement par un dispositif prévu à cet effet ou est lu par l'utilisateur lui-même et doit être entré manuellement dans un dispositif prévu à cet effet.

23. Capsule d'échantillon selon la revendication 18 ou 19, **caractérisée en ce que** ladite capsule comporte un emplacement qui permet de transpercer un septum, par exemple, au moyen d'aiguilles, de tiges, de canules ou dispositifs similaires.

24. Capsule d'échantillon selon la revendication 18 ou 19, **caractérisée en ce que** l'un des éléments de la capsule d'échantillon, qui reste dans le tissu de l'être vivant sur lequel est prélevé l'échantillon, comprend
- un code à barres,
- un circuit intégré, dans lequel on peut lire ou écrire,
- une bande magnétique,
- un transpondeur,
- un émetteur,
- un code à chiffres,
- un code à lettres, ou
- un codage similaire ou système de support d'informations,
- ou un simple repère de couleur ou élément analogue.

25. Capsule d'échantillon selon la revendication 19, **caractérisée en ce que** la marque d'oreille restant dans le tissu peut être retirée uniquement en laissant des dommages visibles soit contre la marque d'oreille, soit sur l'être vivant.

26. Capsule d'échantillon selon la revendication 18 ou 19, **caractérisée en ce que** le capuchon de la capsule d'échantillon, en tant qu'unité de prélèvement d'échantillon, comporte sur le côté opposé à l'échantillon une forure, une fente ou un élément similaire pour la fixation contre un guidage.

27. Capsule d'échantillon selon la revendication 18 ou 19, **caractérisée en ce que** l'élément de la capsule d'échantillon prélevant l'échantillon comporte, sur le côté opposé à l'échantillon, des creux qui sont destinés a) à presser ou serrer l'élément de prélèvement contre le tissu de manière à prélever par pincement un échantillon de tissu, ou b) à guider l'élément de prélèvement en position inclinée et en exerçant une pression le long du tissu de manière à prélever par grattage un échantillon de tissu.

28. Capsule d'échantillon selon la revendication 18 ou 19, **caractérisée en ce que** le capuchon de la capsule d'échantillon, sur le côté opposé à l'échantillon, comporte une saillie permettant de retirer facilement ledit capuchon hors du tube d'échantillon, mais non sans laisser des traces contre la capsule.

29. Capsule d'échantillon selon la revendication 18 ou 19, **caractérisée en ce que** le capuchon de la capsule d'échantillon, en tant que l'élément de prélèvement d'échantillon, comporte sur le côté opposé à l'échantillon un ou plusieurs fentes, forures ou creux destinés à recevoir le tissu.

30. Capsule d'échantillon selon la revendication 18 ou 19, **caractérisée en ce que** le capuchon de la capsule d'échantillon est fendu et est en partie conique, afin que deux ou plusieurs pointes au contact avec le sujet d'échantillage, soient facilement introduites dans celui-ci et, ensuite en raison de la forme conique, soient poussées l'une contre l'autre de manière à pouvoir prélever par grattage ou découpe une faible quantité d'échantillon et aucune matière supplémentaire.

31. Dispositif et capsule d'échantillon selon la revendication 1, 18 ou 19, **caractérisés en ce que** le tube d'échantillon, après fermeture par le capuchon de capsule d'échantillon, ne peut plus être ouvert ou son contenu n'est accessible qu'en laissant subsister des traces visibles.

32. Dispositif selon la revendication 1, **caractérisé en ce que** des tubes d'échantillon individuels ou multiples reliés en chaîne comportent un numéro de série.

33. Dispositif et capsule d'échantillon selon la revendication 1, 18 ou 19, **caractérisés en ce que** des éléments de la capsule d'échantillon sont remplis de réactifs nécessaires pour le traitement ultérieur de l'échantillon.
